# EUROPEAN PATENT APPLICATION

(11) **EP 4 202 063 A1**
(43) Date of publication of application: **28.06.2023**
(21) Application number: 21383198.5
(22) Date of filing: 22.12.2021
(51) Int. Cl.: C12Q 1/70, C12Q 1/6851

(54) **METHOD AND KIT FOR THE DETECTION OF THE SARS-COV-2 VIRUS BASED ON A LOOP-MEDIATED ISOTHERMAL AMPLIFICATION (RT-LAMP) AND OPTICAL AND/OR ELECTROCHEMICAL DETECTION**

(71) Applicant: Fundación Gaiker, 48170 Zamudio Vizcaya (ES); Fundación Tekniker, 20600 Eibar (Guipuzkoa) (ES)
(72) Inventor: OLABARRIA DE PABLO, Garbiñe, 48170 Zamudio (BIZKAIA) (ES); RIVAS MACHO, Ana, 48170 Zamudio (BIZKAIA) (ES); GOÑI DE CERIO, Felipe, 48170 Zamudio (BIZKAIA) (ES); MERINO ALVAREZ, Santos, 20600 Eibar (GIPUZKOA) (ES); DIEZ AHEDO, Ruth, 20600 Eibar (GIPUZKOA) (ES); ELETXIGERRA HERNANDEZ, Unai, 20600 Eibar (GIPUZKOA) (ES)
(74) Representative: Balder IP Law, S.L.

(57) **Abstract**

The present invention provides a method for the detection and/or diagnosis of the SARS-CoV-2 virus (COVID-19), in an isolated biological sample, by means of reverse transcription loop-mediated isothermal amplification (RT-LAMP), comprising the amplification of specific regions of the SARS-CoV-2 virus, using a set of specially designed RT-LAMP primers, and wherein the detection of the amplification products is carried out by means of an optical and/or electrochemical method. The present invention also relates to the set of specially designed RT-LAMP primers, to a reading reagent and to a kit for specifically detecting the amplified sequences of the SARS-CoV-2 virus comprising the set of specially designed RT-LAMP primers according to the present invention.

## Description

### TECHNICAL FIELD

The present invention relates to a method for the detection and/or diagnosis of the SARS-CoV-2 virus (COVID-19) in a sample from a subject, by means of reverse transcription loop-mediated isothermal amplification (RT-LAMP) technology, comprising the amplification of specific regions of the SARS-CoV-2 genome, preferably at least two regions selected from region ND3B and region S.45, of a set of five regions of said virus, using a set of specially designed RT-LAMP primers, and wherein the detection of the absence and/or presence of said amplification products is carried out by means of an optical and/or electrochemical method. The present invention also relates to the set of specially designed RT-LAMP primers for the amplification of the mentioned regions, to a reading reagent for specifically detecting the specific amplified regions of the SARS-CoV-2 virus, and to a kit for specifically detecting the amplified sequences of the virus comprising the set of specially designed RT-LAMP primers according to the present invention.

### STATE OF THE ART

COVID-19 has exposed the serious limitations of society's capabilities to respond to pandemic emergencies, and it has clearly shown that it is necessary to expand and strengthen the portfolio of tools available for the effective diagnosis of infectious diseases both now and in the future. Today, almost 267 million people have been diagnosed with COVID-19 worldwide, and the disease has caused the death of more than 5.2 million people (https://coronavirus.jhu.edu/ data from December 2021), making it the deadliest infectious disease of the century. These figures have clearly shown the recognised need, on an international level, to have rapid and cost-effective tests which allow knowing the degree of extension of the SARS-CoV-2 infection in the population. Thus, knowing said degree of extension of the infection allows the viral transmission to very significantly be contained and aids in making decision for managing the criteria for mitigating the spread of the virus by preventing, to the extent possible, same from representing a greater irreversible destruction of use or an increase in social unrest. According to the publications of the WHO, the diagnostic tests for COVID-19 are critical for tracking the virus, understanding the epidemiology, reporting on the handling of cases and reducing transmission (WHO. 2019).

The real-time quantitative PCR (RT-qPCR) technique is currently the methodology of reference for the detection of COVID-19. Despite its unquestionable precision and robustness, methods based on RT-qPCR have some important limitations such as, for example, lack of portability, dependence on centralised facilities, need for technical knowledge to perform the tests, and high infrastructure and operating costs, which impede offering *in situ* diagnoses that are rapid, cost-effective and massive in the current context brought on by the COVID-19 pandemic.

Hereinafter and throughout the present document, the RT-qPCR technique shall be referred to as PCR, but the present document refers at all times to RT-qPCR.

A potential alternative to RT-qPCR could be the RT-LAMP methodology, since recent reports have documented that the RT-LAMP technique has a sensitivity and specificity comparable to those of RT-qPCR. And most importantly, the RT-LAMP technique offers attractive advantages over RT-qPCR in terms of: (i) simplification of the equipment and reduction of consumption, (ii) no need for an optical module, since detection is possible with the naked eye due to a change in colour, (iii) possibility of transferring the RNA to the amplified product simultaneously, without any need for the two steps of RT and PCR, (iv) greater resistance to the presence of inhibitors from the sample, and (v) high amplification yield, high specificity and high sensitivity. All this translated into lower costs and capital and operations, combined with a high amplification yield and a high specificity and sensitivity.

However, colorimetric RT-LAMP methods also have some limitations. One of them is based on the fact that the difference in colour can be negligible when the initial amount of RNA is very low and that certain dyes produce a partial inhibition of the enzyme polymerase when the amount of starting RNA is less than 100 absolute viral copies in the reaction, thereby negatively affecting the detection of the virus in clinical samples with a very low viral load.

Therefore, there is still a need in the state of the art to have new *Point* Of *Care* (POC) methods and systems available for the detection of subjects with COVID-19 which are sensitive, precise, rapid and low-cost for identifying, outside of specialised laboratories and in the same locations where samples are taken, infected individuals, facilitating the suitable isolation thereof, and helping to control the spread of the disease.

### DESCRIPTION OF THE INVENTION

To solve the problems existing in the state of the art, the present invention has developed a POC method and a system or kit for the detection of subjects with COVID-19 based on an isothermal amplification and subsequent optical detection, preferably by means of colorimetry and/or fluorimetry, and/or electrochemical detection, of the amplified products, where the result of the amplification is or can be directly observed with the naked eye. Said POC method and kit is based on an amplification of the SARS-CoV-2 viral RNA by means of RT-LAMP, more specifically, the methods and kits of the present invention are based on the amplification of specific regions of the SARS-CoV-2 genome, by means of a set of specially designed RT-LAMP primers for the identification of said regions of the viral RNA, and the subsequent detection of the presence of said amplified regions by means of a colorimetric, fluorometric and/or electrochemical detection system.

Hereinafter and throughout the present document, both the RT-LAMP technique and the primers used in said technique shall be referred to as LAMP "technique/method" and "LAMP primers", but the present document refers at all times to the RT-LAMP technique/method, and to the primers for said RT-LAMP technique/method.

The possibility of detecting the results of the LAMP amplification with an electrochemical detection system provides a significant improvement in the detection of SARS-CoV-2 by means of optical detection, said system allowing a rapid, simple, more sensitive and more precise detection than that used up until now with the known methods. Thus, the present invention proposes, in addition to an optical detection system having a very high sensitivity and specificity in comparison with other SARS-CoV-2 optical detection systems used in the art, providing an electrochemical detection and reading system, the advantage of which over the optical detection system is that it provides a numerical value, which is more objective that viewing the difference in colour with the naked eye provided by the optical detection and reading system, and furthermore, said electrochemical system is completely adaptable to a low-cost portable system that uses an electrochemical reading technology widely used on the market for other applications.

Thus, the POC system described in the present description will allow the healthcare system to have better control over and prevention of the SARS-CoV-2 virus for the following reasons: (i) improved early control of SARS-CoV-2 by means of the application of an alternative detection tool based on a genetic method of isothermal amplification, LAMP, combined with electrochemical detection; (ii) detection of SARS-CoV-2 without any need for sophisticated laboratory equipment; (iii) ease in the interpretation of the results with electrochemical detection compared to existing methods which require sophisticated optical readers for the detection of the fluorescence emission; (iv) significant shortening of detection times with respect to RT-qPCR for the purpose of streamlining decision making concerning the confinement of the carriers and (v) a reduction in the cost in consumables with respect to the RT-qPCR system.

Thus, in a first aspect, the present description relates to an *in vitro* and/or ex *vivo* method for the detection of the SARS-CoV-2 virus in an isolated biological sample, hereinafter referred to as the first method of the invention, wherein the method comprises the steps of:
a) extracting genetic material from the isolated biological sample;
b) performing RT-LAMP isothermal amplification of the genetic material isolated in step (a), with a reading reagent comprising at least one amplification solution, said amplification solution comprises a set of LAMP primers for amplifying at least one specific region of the SARS-CoV-2 virus selected from: region ND1-1 (SEQ ID NO: 1), region ND3B (SEQ ID NO: 2), region S.45 (SEQ ID NO: 3), region ORF1ab.99 (SEQ ID NO: 4), region E.105 (SEQ ID NO: 5), and/or any of the combinations thereof, preferably at least two specific regions of the SARS-CoV-2 virus selected from: region ND3B (SEQ ID NO: 2) and region S.45 (SEQ ID NO: 3);
   wherein the set of LAMP primers for amplifying region ND3B (SEQ ID NO: 2) comprises:
      - a forward primer F3 comprising a nucleotide sequence of SEQ ID NO: 12,
      - a backward primer B3 comprising a nucleotide sequence of SEQ ID NO: 13,
      - a forward inner primer FIP comprising a nucleotide sequence of SEQ ID NO: 14,
      - a backward inner primer BIP comprising a nucleotide sequence of SEQ ID NO: 15,
      - a forward loop primer LF comprising a nucleotide sequence of SEQ ID NO: 16; and
      - a backward loop primer LB comprising a nucleotide sequence of SEQ ID NO: 17;
   and wherein the set of LAMP primers for amplifying region S.45 (SEQ ID NO: 3) in step (b) comprises:
      - a forward primer F3 comprising a nucleotide sequence of SEQ ID NO: 18,
      - a backward primer B3 comprising a nucleotide sequence of SEQ ID NO: 19,
      - a forward inner primer FIP comprising a nucleotide sequence of SEQ ID NO. 20,
      - a backward inner primer BIP comprising a nucleotide sequence of SEQ ID NO: 21,
      - a forward loop primer LF comprising a nucleotide sequence of SEQ ID NO: 22, and
      - a backward loop primer LB comprising a nucleotide sequence of SEQ ID NO: 23,
c) reading and detecting the presence or absence of an amplification product of the specific regions of step b) by means of an optical and/or electrochemical method.

In a second aspect, the present description relates to an *in vitro* and/or ex *vivo* method for the diagnosis of the SARS-CoV-2 virus, and, therefore, for the diagnosis of COVID-19, in an isolated biological sample, hereinafter referred to as second method of the invention, wherein the method comprises the steps of:
a) extracting genetic material from the isolated biological sample;
b) performing loop-mediated isothermal amplification (LAMP) of the genetic material isolated in step (a), with a detection reagent comprising at least one amplification solution, said amplification solution comprises a set of LAMP primers for amplifying at least one specific region of the SARS-CoV-2 virus selected from: region ND1-1 (SEQ ID NO: 1), region ND3B (SEQ ID NO: 2), region S.45 (SEQ ID NO: 3), region ORF1ab.99 (SEQ ID NO: 4), region E.105 (SEQ ID NO: 5), and/or any of the combinations thereof, preferably at least two specific regions of the SARS-CoV-2 virus selected from: region ND3B (SEQ ID NO: 2) and region S.45 (SEQ ID NO: 3);
   wherein the set of LAMP primers for amplifying region ND3B (SEQ ID NO: 2) comprises:
      - a forward primer F3 comprising a nucleotide sequence of SEQ ID NO: 12,
      - a backward primer B3 comprising a nucleotide sequence of SEQ ID NO: 13,
      - a forward inner primer FIP comprising a nucleotide sequence of SEQ ID NO: 14,
      - a backward inner primer BIP comprising a nucleotide sequence of SEQ ID NO: 15,
      - a forward loop primer LF comprising a nucleotide sequence of SEQ ID NO: 16; and
      - a backward loop primer LB comprising a nucleotide sequence of SEQ ID NO: 17;
   and wherein the set of LAMP primers for amplifying region S.45 (SEQ ID NO: 3) comprises:
      - a forward primer F3 comprising a nucleotide sequence of SEQ ID NO: 18,
      - a backward primer B3 comprising a nucleotide sequence of SEQ ID NO: 19,
      - a forward inner primer FIP comprising a nucleotide sequence of SEQ ID NO. 20,
      - a backward inner primer BIP comprising a nucleotide sequence of SEQ ID NO: 21,
      - a forward loop primer LF comprising a nucleotide sequence of SEQ ID NO: 22, and
      - a backward loop primer LB comprising a nucleotide sequence of SEQ ID NO: 23,
c) reading and detecting the presence or absence of an amplification product of the specific regions of step b) by means of an optical and/or electrochemical method,
   wherein
   i. the presence of amplification product for both regions ND3B and S.45 is indicative of the presence of SARS-CoV-2 in the sample, and
   ii. the absence of amplification product for both regions ND3B and S.45 is indicative of the absence of SARS-COV-2 in the sample.

In a third aspect, the present description relates to a set of LAMP primers for amplifying at least one specific region of the SARS-CoV-2 virus selected from: region N D1-1 (SEQ ID NO: 1), region ND3B (SEQ ID NO: 2), region S.45 (SEQ ID NO: 3), region ORF1ab.99 (SEQ ID NO: 4), region E.105 (SEQ ID NO: 5), and/or any of the combinations thereof, preferably at least two regions of the SARS-CoV-2 viruses selected from: region ND3B (SEQ ID NO: 2) and region S.45 (SEQ ID NO: 3); hereinafter referred to as the set of LAMP primers of the invention, wherein the set of LAMP primers for amplifying region ND3B (SEQ ID NO: 2) comprises:
- a forward primer F3 comprising a nucleotide sequence of SEQ ID NO: 12,
- a backward primer B3 comprising a nucleotide sequence of SEQ ID NO: 13,
- a forward inner primer FIP comprising a nucleotide sequence of SEQ ID NO: 14,
- a backward inner primer BIP comprising a nucleotide sequence of SEQ ID NO: 15,
- a forward loop primer LF comprising a nucleotide sequence of SEQ ID NO: 16; and
- a backward loop primer LB comprising a nucleotide sequence of SEQ ID NO: 17;
and wherein the set of LAMP primers for amplifying region S.45 (SEQ ID NO: 3) comprises:
- a forward primer F3 comprising a nucleotide sequence of SEQ ID NO: 18,
- a backward primer B3 comprising a nucleotide sequence of SEQ ID NO: 19,
- a forward inner primer FIP comprising a nucleotide sequence of SEQ ID NO. 20,
- a backward inner primer BIP comprising a nucleotide sequence of SEQ ID NO: 21,
- a forward loop primer LF comprising a nucleotide sequence of SEQ ID NO: 22, and
- a backward loop primer LB comprising a nucleotide sequence of SEQ ID NO: 23.

In a fourth aspect, the present invention relates to the use of the LAMP primers of the present invention for detecting the presence of the SARS-CoV-2 virus and/or for the diagnosis of the SARS-CoV-2 virus, in an isolated biological sample, preferably by means of the methods described in the present invention.

In a fifth aspect, the present description relates to a reading reagent for detecting at least one specific region of the SARS-CoV-2 virus selected from: region N D1-1 (SEQ ID NO: 1), region ND3B (SEQ ID NO: 2), region S.45 (SEQ ID NO: 3), region ORF1ab.99 (SEQ ID NO: 4), region E.105 (SEQ ID NO: 5), and/or any of the combinations thereof, preferably at least two regions of the SARS-CoV-2 virus selected from: region ND3B (SEQ ID NO: 2) and region S.45 (SEQ ID NO: 3), hereinafter referred to as reading reagent of the present invention, wherein said reading reagent comprises at least one amplification solution, wherein said amplification solution comprises the set of LAMP primers of the present invention.

In a sixth aspect, the present invention relates to the use of the reading reagent of the present invention for detecting the presence of the SARS-CoV-2 virus and/or for the diagnosis of the SARS-CoV-2 virus, in an isolated biological sample, preferably by means of the methods described in the present invention.

In a seventh aspect, the present description relates to a kit, device, biosensor or POC, where any of said terms will be used interchangeably throughout the present invention, for the detection and/or diagnosis of the SARS-CoV-2 virus in an isolated biological sample, hereinafter referred to as kit, device or POC of the present invention, wherein said kit, device or POC comprises reagents for amplifying the genetic material in said sample using a LAMP technique and the set of LAMP primers of the present invention and/or the reading reagent of the present invention.

In an eighth aspect, the present description relates to use of the kit, biosensor, device or POC of the present invention, for the detection of the SARS-CoV-2 virus and/or for the diagnosis of COVID-19, in an isolated biological sample.

### BRIEF DESCRIPTION OF THE FIGURES

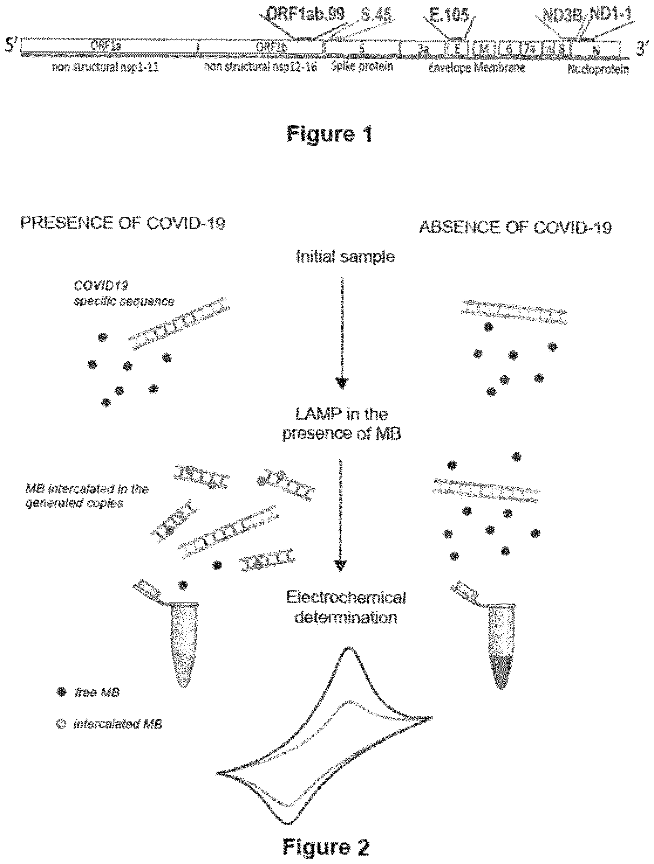

**Figure 1****.** Location of the regions for the LAMP isothermal amplification in the SARS-CoV-2 viral RNA.
**Figure 2****.** Outline of the protocol for the detection of SARS-CoV-2 by means of LAMP isothermal amplification and electrochemical detection. MB: methylene blue.

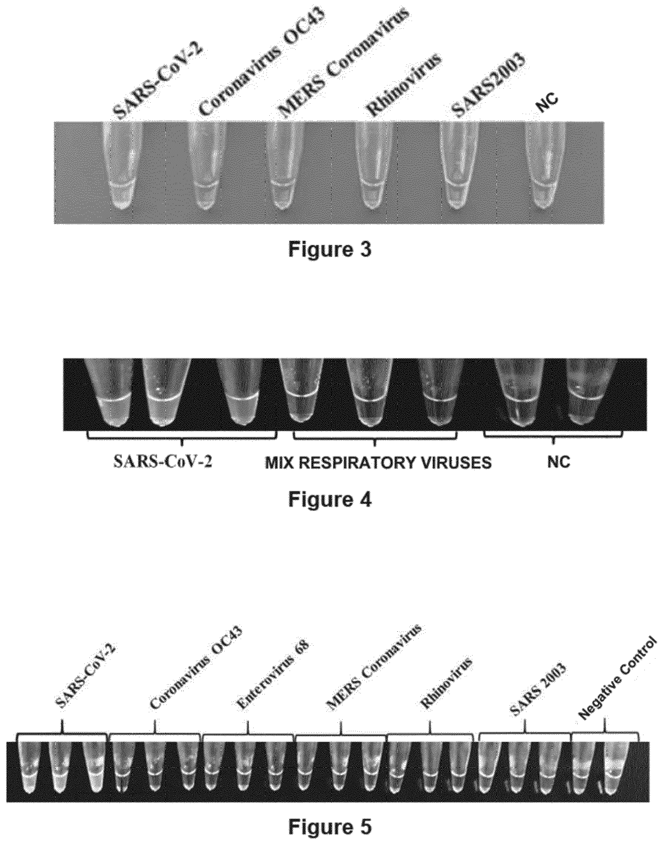

**Figure 3****.** Colorimetric results obtained in the specificity tests with 1,000 RNA copies of the inclusive and exclusive viruses for region ND1-1 (SEQ ID NO: 1) of SARS-CoV-2. It can be seen how the SARS-CoV-2 sample (first tube) has a colour (green) that is completely different from the colour (orange) of the samples comprising the exclusive viruses (tubes 2 to 5) detailed in **Table 2.** NC: negative control (tube 6).
**Figure 4****.** Colorimetric results obtained in the specificity tests with the RNA of the SARS-CoV-2 virus and of the panel of respiratory viruses in **Table** 2 for region ND1-1 (SEQ ID NO: 1) of SARS-CoV-2. It can be seen how the SARS-CoV-2 sample (the three first tubes) has a colour (dark green) that is completely different from the colour (pallid and almost transparent) of the samples (tubes 3 to 6) comprising the respiratory viruses detailed in **Table** 2. NC: negative control (tubes 7 to 8).
**Figure 5****.** Colorimetric results obtained in the specificity tests with 1,000 RNA copies of the inclusive and exclusive viruses for region ND3B (SEQ ID NO: 2) of SARS-CoV-2. It can be seen how the SARS-CoV-2 sample (tubes 1 to 3) has a colour (dark green) that is completely different from the colour (pallid and almost transparent) of the samples comprising the exclusive viruses (tubes 4 to 18) detailed in **Table** 2. NC: negative control (tubes 19 to 20).

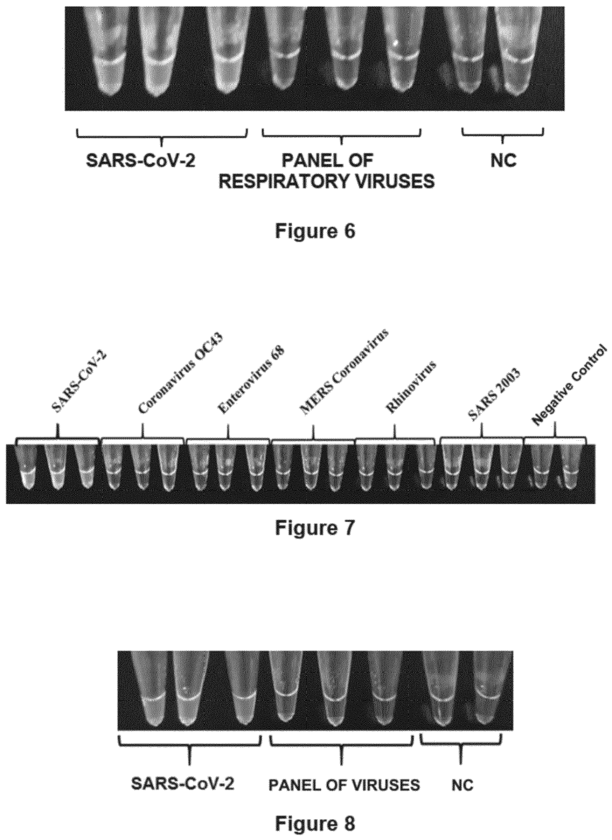

**Figure 6****.** Colorimetric results obtained in the specificity tests with the RNA of the SARS-CoV-2 virus and of the panel of respiratory viruses in **Table 2** for region ND3B (SEQ ID NO: 2) of SARS-CoV-2. It can be seen how the SARS-CoV-2 sample (tubes 1 to 3) has a colour (dark green) that is completely different from the colour (pallid and almost transparent) of the samples comprising the respiratory viruses (tubes 4 to 6) detailed in **Table 2** and the negative control (NC).
**Figure 7****.** Colorimetric results obtained in the specificity tests with 1,000 RNA copies of the inclusive and exclusive viruses for region S.45 (SEQ ID NO: 3) of SARS-CoV-2. It can be seen how the SARS-CoV-2 sample (tubes 1 to 3) has a colour (dark green) that is completely different from the colour (pallid and almost transparent) of the samples comprising the exclusive viruses detailed (tubes 4 to 18) in **Table 2** and the negative control (NC) (tubes 19 to 20).
**Figure 8****.** Colorimetric results obtained in the specificity tests with the RNA of the SARS-CoV-2 virus and of the panel of respiratory viruses in **Table 2** for region S.45 (SEQ ID NO: 3) of SARS-CoV-2. It can be seen how the SARS-CoV-2 sample (tubes 1 to 3) has a colour (dark green) that is completely different from the colour (pallid and almost transparent) of the samples comprising the respiratory viruses (tubes 4 to 6) detailed in the **Table 2** and the negative control (NC) (tubes 7 to 8).

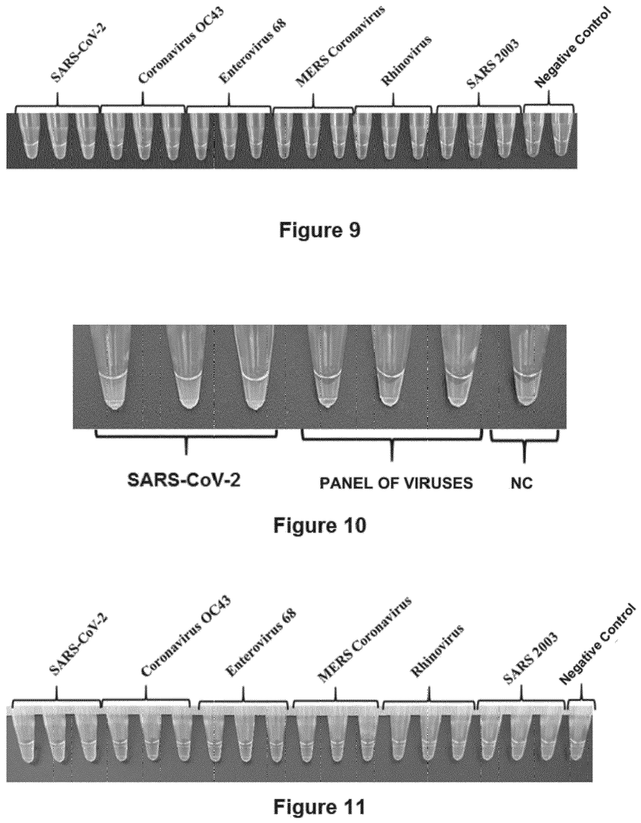

**Figure 9****.** Colorimetric results obtained in the specificity tests with 1,000 RNA copies of the inclusive and exclusive viruses for region ORF1ab.99 (SEQ ID NO: 4) of SARS-CoV-2. It can be seen how the SARS-CoV-2 sample (tubes 1 to 3) has a colour (dark green) that is completely different from the colour (pallid and almost transparent) of the samples comprising the exclusive viruses (tubes 4 to 18) detailed in **Table 2** and the negative control (NC) (tubes 19 to 20).
**Figure 10****.** Colorimetric results obtained in the specificity tests with the RNA of the SARS-CoV-2 virus and of the panel of respiratory viruses in **Table 2** for region ORF1ab.99 (SEQ ID NO: 4) of SARS-CoV-2. It can be seen how the SARS-CoV-2 sample (tubes 1 to 3) has a colour (dark green) that is completely different from the colour (pallid and almost transparent) of the samples comprising the respiratory viruses (tubes 4 to 6) detailed in **Table 2** and the negative control (NC) (tube 7).
**Figure 11****.** Colorimetric results obtained in the specificity tests with 1,000 RNA copies of the inclusive and exclusive viruses for region E.105 (SEQ ID NO: 5) of SARS-CoV-2. It can be seen how the SARS-CoV-2 sample (tubes 1 to 3) has a colour (dark green) that is completely different from the colour (pallid and almost transparent) of the samples comprising the exclusive viruses (tubes 4 to 18) detailed in **Table 2.** NC: negative control (tube 19).

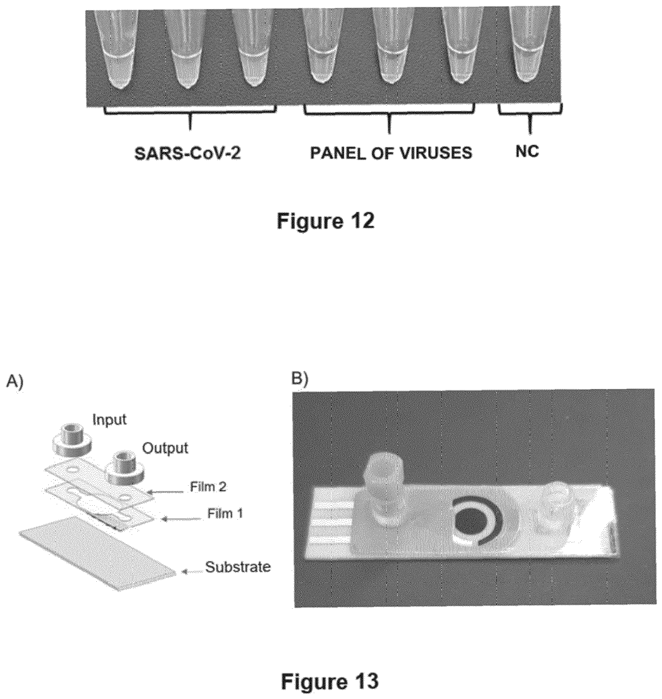

**Figure 12****.** Colorimetric results obtained in the specificity tests with the RNA of the SARS-CoV-2 virus and of the panel of respiratory viruses in **Table 2** for region E.105 (SEQ ID NO: 5) of SARS-CoV-2. It can be seen how the SARS-CoV-2 sample (tubes 1 to 3) has a colour (dark green) that is completely different from the colour (pallid and almost transparent) of the samples comprising the respiratory viruses (tubes 4 to 6) detailed in **Table 2** and the negative control (NC) (tube 7).
**Figure 13. A****)** Diagram for the assembly of the biosensor prototype of the present invention for the electrochemical detection of SARS-CoV-2. **B****)** Assembly of the electrochemical biosensor prototype of the present invention on a commercial electrochemical detection chip.

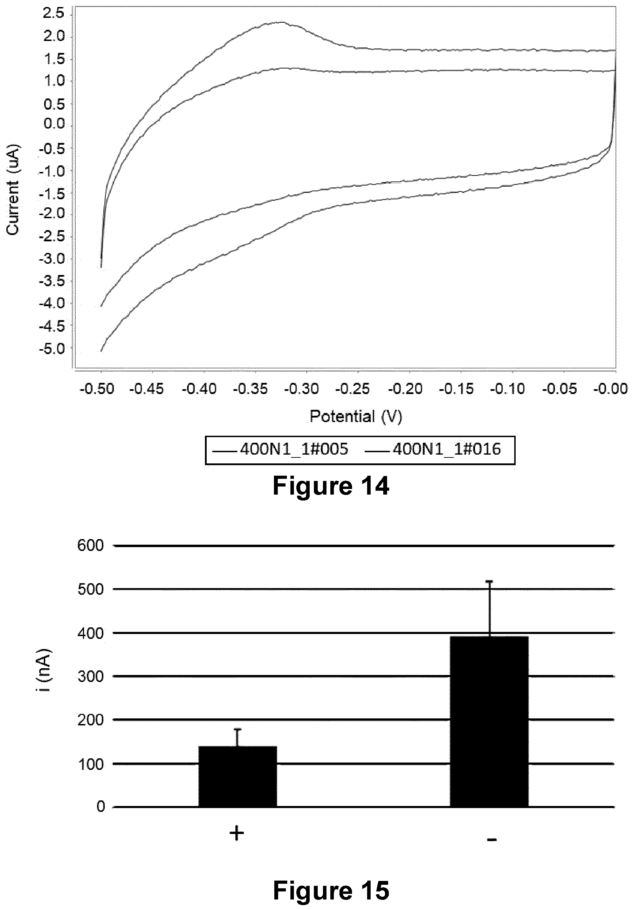

**Figure 14****.** Cyclic voltammetries of samples positive and negative for SARS-CoV-2 obtained by means of the electrochemical detection method of the present invention.
**Figure 15****.** Graph showing electrochemical current intensity values of the oxidation peak obtained by cyclic voltammetry for positive and negative SARS-CoV-2 samples by means of the method of the present invention. The error bars indicate the standard deviation for three repetitions.

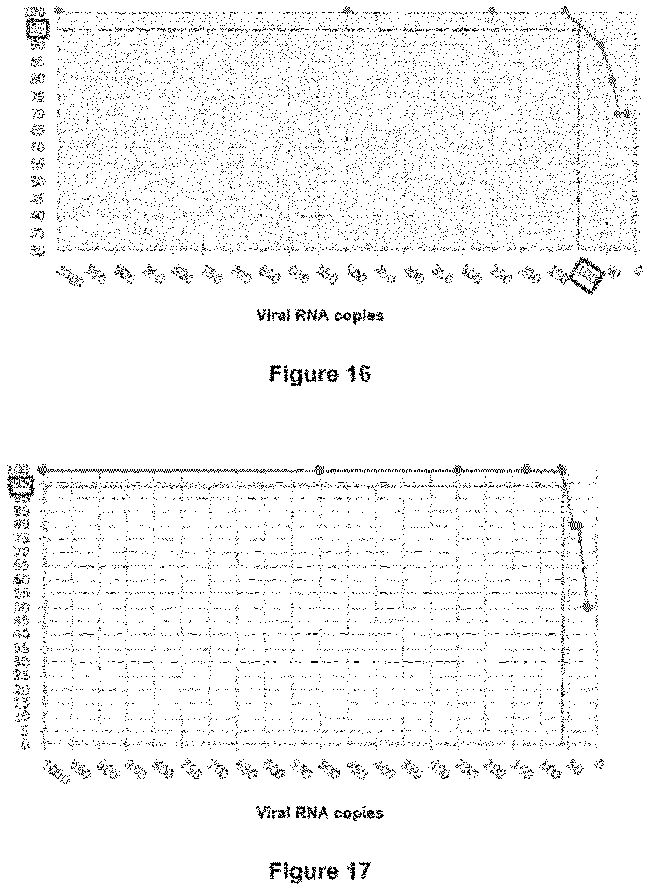

**Figure 16****.** Results of the limit of detection obtained for region ND1-1 (SEQ ID NO: 1). The viral copies vs the positivity percentage per each level of viral copies analysed are represented.
**Figure 17****.** Results of the limit of detection obtained for region ND3B (SEQ ID NO: 2). The viral copies vs the positivity percentage per each level of viral copies analysed are represented.

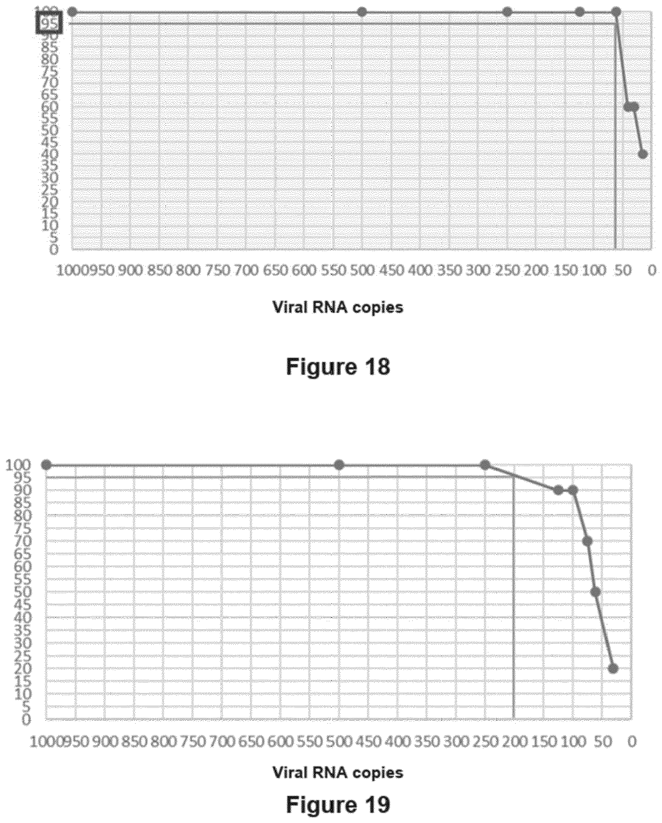

**Figure 18****.** Results of the limit of detection obtained for region S.45 (SEQ ID NO: 3). The viral copies vs the positivity percentage per each level of viral copies analysed are represented.
**Figure 19****.** Results of the limit of detection obtained for region ORF1ab.99 (SEQ ID NO: 4). The viral copies vs the positivity percentage per each level of viral copies analysed are represented.

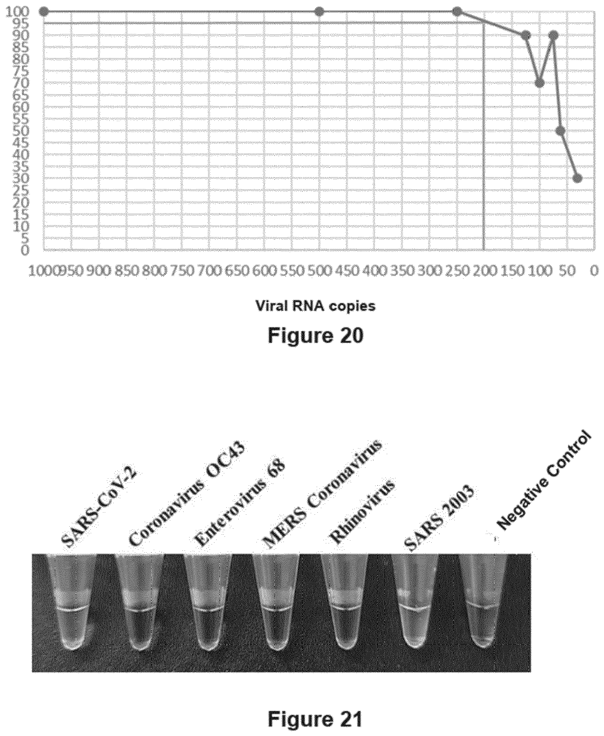

**Figure 20****.** Results of the limit of detection obtained for region E.105 (SEQ ID NO: 5). The viral copies vs the positivity percentage per each level of viral copies analysed are represented.
**Figure 21****.** Colorimetric results obtained with the SARS-CoV-2 Rapid colorimetric LAMP kit (New England Biolabs) in the specificity tests with 1,000 RNA copies of the inclusive and exclusive viruses in **Table 2.** Positive results for SARS-CoV-2 and SARS 2003 (tubes 1 and 6) are shown in yellow, and negative results for the rest of the viruses in **Table 2** analysed (tubes 2 to 5 and negative control) are shown in pink.

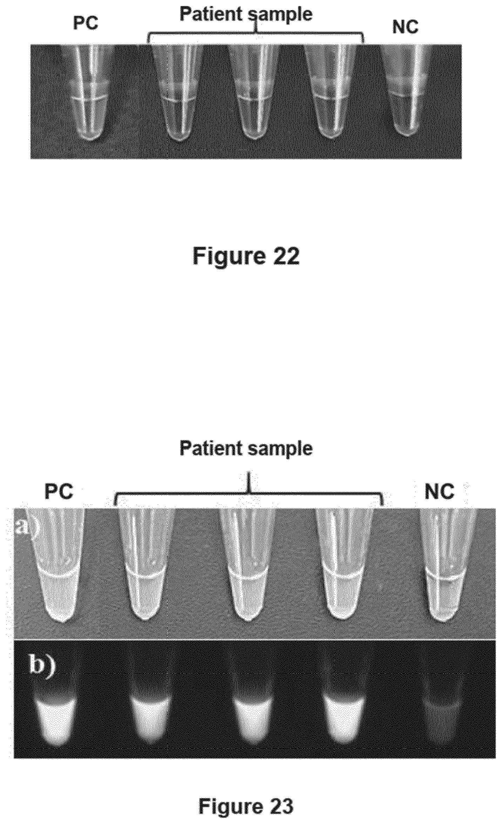

**Figure 22****.** Colorimetric results obtained with the SARS-CoV-2 Rapid colorimetric LAMP kit (New England Biolabs) in the analytical tests of a clinical sample in which SARS-CoV-2 RNA (doped sample) has been included and assayed in triplicate. In yellow se has the Positive results (first tube) are shown in yellow, and negative results (tubes 2 to 4 and the negative control (NC)) are shown in pink.
**Figure 23****.** Colorimetric results **(a)** obtained with the method of the present invention for region S.45 (SEQ ID NO: 3) of SARS-CoV-2 in a clinical sample to which SARS-CoV-2 RNA (doped RNA sample) has been added and assayed in triplicate. Dark yellow **(a)** shows the positive results (tubes 1 to 4 which refer to a PC: positive control and the sample from the patient in triplicate) and pale orange shows the negative result (tube 4 NC: negative control). Part **(b)** shows the same tubes but analysed in a UV transilluminator where the positive results show a fluorescence emission with respect to the negative result.

### DETAILED DESCRIPTION OF THE INVENTION.

The following definitions and methods are provided to better define the present invention and to guide those skilled in the art in the practice thereof. Unless indicated otherwise, the terms must be understood according to the conventional use of those skilled in the art. Each aspect thus defined can be combined with any other aspect or aspects, unless clearly indicated otherwise. Particularly, any feature indicated as preferred or advantageous can be combined with any other feature or features indicated as preferred or advantageous.

The practice of the present invention shall employ, unless indicated otherwise, conventional molecular biology, chemistry, biochemistry techniques and bioinformatic and recombinant DNA technology that are within the capabilities of one skilled in the art. Said techniques are explained extensively in the literature.

In a first aspect, the present description relates to an *in vitro* and/or ex *vivo* method for the detection of the SARS-CoV-2 virus in an isolated biological sample, first method of the invention, wherein the method comprises the steps of:
a) extracting genetic material from the isolated biological sample;
b) performing loop-mediated isothermal amplification (LAMP) of the genetic material isolated in step (a), with a reading reagent comprising at least one amplification solution, said amplification solution comprises a set of LAMP primers for amplifying at least one specific region of the SARS-CoV-2 virus selected from: region N D1-1 (SEQ ID NO: 1), region ND3B (SEQ ID NO: 2), region S.45 (SEQ ID NO: 3), region ORF1ab.99 (SEQ ID NO: 4), region E.105 (SEQ ID NO: 5), and/or any of the combinations thereof, preferably at least two specific regions of the SARS-CoV-2 virus preferably selected from region ND3B (SEQ ID NO: 2) and region S.45 (SEQ ID NO: 3);
   wherein the set of LAMP primers for amplifying region ND3B (SEQ ID NO: 2) comprises:
      - a forward primer F3 comprising a nucleotide sequence of SEQ ID NO: 12,
      - a backward primer B3 comprising a nucleotide sequence of SEQ ID NO: 13,
      - a forward inner primer FIP comprising a nucleotide sequence of SEQ ID NO: 14,
      - a backward inner primer BIP comprising a nucleotide sequence of SEQ ID NO: 15,
      - a forward loop primer LF comprising a nucleotide sequence of SEQ ID NO: 16; and
      - a backward loop primer LB comprising a nucleotide sequence of SEQ ID NO: 17;
   and wherein the set of LAMP primers for amplifying region S.45 (SEQ ID NO: 3) comprises:
      - a forward primer F3 comprising a nucleotide sequence of SEQ ID NO: 18,
      - a backward primer B3 comprising a nucleotide sequence of SEQ ID NO: 19,
      - a forward inner primer FIP comprising a nucleotide sequence of SEQ ID NO. 20,
      - a backward inner primer BIP comprising a nucleotide sequence of SEQ ID NO: 21,
      - a forward loop primer LF comprising a nucleotide sequence of SEQ ID NO: 22, and
      - a backward loop primer LB comprising a nucleotide sequence of SEQ ID NO: 23,
c) reading and detecting the presence or absence of an amplification product of the specific regions of step b) by means of an optical and/or electrochemical method.

In the present invention, the term *"in vitro"* and *"ex vivo"* refer to the fact that the method of the invention is performed outside of the subject's body.

For purposes of the present invention, the term "subject", "individual" and "patient" are used interchangeably. Said terms refer to all the animals classified as mammals and includes, but it is not restricted to, domestic and farm animals, primates and humans, for example, human beings, non-human primates, cows, horses, pigs, sheep, goats, dogs, cats, or rodents. Preferably, the subject is a human male or female of any age or race.

The term "biological sample" in the present invention refers to any sample which allows the amplification products of the specific sequences of the SARS-CoV-2 virus in said sample to be detected and/or quantified. Thus, the term biological sample, includes but is not limited to environmental samples, such as water samples, preferably a clean water sample, waste water sample or air samples, and any biological fluid sample from an individual, obtained by means of any method known to one skilled in the art serving such purpose. The biological sample comprises RNA and/or protein, preferably messenger RNA. The biological sample could be, for example, but is not limited to, a nasopharyngeal sample or a saliva sample. Preferably, the biological sample is a nasopharyngeal sample.

The biological samples and associated data of the subjects included in the present invention were collected, processed and transferred by the *Biobanco Vasco* (Basque Biobank)/Nodo *BioCruces Bizkaia* (Bizkaia BioCrossing Node) www.biobancovasco.org following standard operating procedures and with the approval of the Ethics Committee and the Scientific Committee.

In the present invention, the term "genetic material" refers to nucleic acid, nucleic acid sequence, nucleic acid molecule, etc. As they are used in this document, the words "nucleic acid", "nucleic acid sequence", "nucleotide", "nucleic acid molecule" or "polynucleotide" intend to include DNA molecules (for example, cDNA or genomic DNA), RNA molecules (for example, mRNA), natural, mutated and synthetic DNA or RNA molecules, and DNA or RNA analogues generated by means of nucleotide analogues. They can be single-stranded or double-stranded. Said nucleic acids or polynucleotides include, but are not limited to, coding sequences of structural genes, antisense sequences and non-coding regulatory sequences that do not code mRNA or protein products. In a preferred embodiment, the terms genetic material and nucleic acid, as well as the different meanings thereof, preferably refer to RNA. These terms also include a gene. The term "gene" or "genetic sequence" is used in a broad sense to refer to a DNA nucleic acid associated with a biological function. Thus, the genes can include introns and exons like in the genomic sequence, or they can comprise only one coding sequence like in cDNA, and/or they can include cDNA in combination with regulatory sequences.

In a preferred embodiment of the first method of the invention, step a) of the method comprises extracting genetic material from the isolated biological sample, wherein said genetic material is preferably RNA.

In another preferred embodiment of step a) of the first method of the present invention, the extraction of the genetic material, preferably nucleic acid, and more preferably RNA, is carried out by means of any commercial product used in the art for such purpose. By way of example, the following commercial kits can be used in the present invention, Quick-RNA^{™} Viral Kit (ZYMO RESEARCH); Dynabeads^{®} SILANE viral NA (Thermofisher); NucleoMag Pathogen Kit (MACHEREY-NAGEL) and MagMaxTM Viral/Pathogen Nucleic Acid Isolation Kit (Applied biosystems), although any other also can be useful if it is designed for the same purpose.

In another more preferred embodiment of the first method of the present invention, the extraction of said genetic material in step a) is carried out, preferably by means of two different extraction systems or methods, a first rapid extraction system or method, based on the use of a commercial reagent and a thermostat preferably for the detection of symptomatic subjects, and a second extraction system or method comprising magnetic microparticles and preferably used for population screening, in general, particularly for use in asymptomatic subjects.

In another more preferred embodiment, the rapid extraction method preferably for the detection of symptomatic subjects can be carried out in turn by means of two techniques, the first of said techniques, which shall be referred to as "Option A" for purposes of the present invention, is a technique in which a commercial kit and a thermostat are used, preferably the commercial QuickExtract^{™} DNA Extraction Solution kit (Lucigen) has been used in the examples included in the present invention, although any other kit known to one skilled in the art can be used. By means of said Option A, it is therefore necessary to extract the RNA from the sample with the mentioned kit, and said RNA is subsequently incubated with the amplification solution to subsequently carry out optical and/or electrochemical detection.

Furthermore, the rapid extraction method preferably for the detection of symptomatic subjects can also be carried out by means of "Option B" which is a more rapid technique than Option A since the prior step of extracting the RNA from the sample is not required. By means of Option B, the biological sample is mixed directly with the amplification solution, wherein said amplification solution preferably comprises at least 25 units of RNAse inhibitor enzyme.

Both Option A and Option B can be used interchangeably in the present invention. In addition, both the rapid extraction method preferably for the detection of symptomatic subjects and the extraction method preferably for population screening can be used interchangeably in the present invention for the detection and/or diagnosis of SARS-CoV-2 in an isolated biological sample, both for symptomatic subjects and for a population analysis, although for purposes of the present invention, Option B is preferable for the extraction of the genetic material in symptomatic cases because of how rapid it is.

In a more preferred embodiment of the first method of the present invention, said method is characterised in that the rapid extraction method for extracting the nucleic acid from the isolated biological sample preferably used for the case of symptomatic subjects, and more specifically, Option A comprises the following steps:
1. Dispensing the isolated biological sample in a tube with a lysis reagent, for purposes of the present invention, the lysis reagent used in the examples is the Quick Extract kit (Lucigen) at an approximate ratio of 1:1, and mixing.
2. Incubating the sample of step 1. for at least 5 min at a temperature of at least 95 °C.
3. Adding a volume to the amplification mixture which will be described in detail below and continuing with the steps described in the first and second method of the present invention.

In another more preferred embodiment of the first method of the present invention, said method is characterised in that the rapid extraction method for extracting the nucleic acid from the isolated biological sample preferably used for the case of symptomatic subjects, and more specifically, the Option B, comprises the following steps:
1. Dispensing the isolated biological sample in a tube comprising the amplification solution according to the present invention. In a more preferred embodiment, the amplification sample comprises at least 25 units of RNAse inhibitor enzyme.
2. Continuing with the steps described in the first and second method of the present invention.

In another more preferred embodiment of the first method of the present invention, said method is characterised in that the extraction method for extracting the nucleic acid, preferably for population screening, comprises magnetic microparticles and is preferably used for population screening. Reagents of the Applied Biosystems^{™} MagMAX^{™} Viral/Pathogen Nucleic Acid Isolation Kit are used in this step, and the manufacturer's instructions are followed, although the methodology has been subjected to adaptations so that the extraction process for extracting nucleic acid from the sample is simpler, rapid and with a higher extraction yield. By means of the optimised system described in the present invention, it is possible to extract the volume samples required by the user by scaling the rest of the volumes of the process. Once the nucleic acid has been extracted by means of the described technology, it is mixed with the amplification mixture which will be described in detail below, and the method continues, following the steps described for the first method of the present invention. Although Applied Biosystems^{™} MagMAX^{™} Viral/Pathogen Nucleic Acid Isolation Kit has been used in the examples included in the present invention, any other commercial kit known in the art and used for the same purpose can be used in the present invention.

In another preferred embodiment of the first method of the present invention, said method is based on RT-LAMP technology and, accordingly, isothermal amplification (constant temperature) of specific regions of the SARS-CoV-2 virus genome is carried out. This allows the molecular assay to be carried out at a single temperature without the requisite temperature cycles.

Another achievement of the method according to the present invention is that the reading reagent of the invention comprising the amplification solution allows the steps of reverse transcription, amplification and detection to be carried out simultaneously and in the same place, reaching a limit of detection of 62 viral copies for any of the optical and/or electrochemical detection methods.

For the design of the isothermal amplification, first the decision was made to select the specific regions of the SARS-CoV-2 virus genome that were going to be used and for which the LAMP primers **(****Figure 1****)** would subsequently be designed. Thus, the different regions of the SARS-CoV-2 genome, described with GenBank (NCBI) accession number NC_045512.2, and the following are used in the present invention:
- Region ND1-1: is a region found in the nucleotide sequence of the *N* gene coding for the protein of the virus nucleoprotein. The nucleotide sequence of the N gene is described under GenBank (NCBI) accession number NC_045512.2:28,274-29,533. For purposes of the present invention, region ND1-1 comprises the nucleotide sequence defined as SEQ ID NO: 1. Said SEQ ID NO: 1 refers to positions 28,285-28,487 under GenBank accession number NC_045512.2. SEQ ID NO: 1 is represented by the following nucleotide sequence: 
tggaccccaaaatcagcgaaatgcaccccgcattacgtttggtggaccctcagattcaactggcagtaa
ccagaatggagaacgcagtggggcgcgatcaaaacaacgtcggccccaaggtttacccaataatact
 gcgtcttggttcaccgctctcactcaacatggcaaggaagaccttaaattccctcgaggacaaggc.
- Region ND3B: is an intergenic region located between SARS-CoV-2 ORF8 and N genes. The nucleotide sequence of ORF8 gene is described under GenBank (NCBI) accession number NC_045512.2:27894-28259, and the sequence of N gene is described, as previously mentioned, under GenBank (NCBI) accession number NC_045512.2:28,274-29,533. For purposes of the present invention, region ND3B comprises the nucleotide sequence defined as SEQ ID NO: 2 in the present invention, and it is located between positions 28146-28374 of the SARS CoV-2 genome described under NC_045512.2. Said SEQ ID NO: 2 is represented by the following nucleotide sequence: 
attcagtacatcgatatcggtaattatacagtttcctgtttaccttttacaattaattgccaggaacctaaattg
ggtagtcttgtagtgcgttgttcgttctatgaagactttttagagtatcatgacgttcgtgttgttttagatttcatct
aaacgaacaaactaaaatgtctgataatggaccccaaaatcagcgaaatgcaccccgcattacgtttg
gtggaccctcagattcaactggcagtaaccagaatggagaacgcagtggggcgcgatc.
- Region S.45: as it is herein used, said region can be found in the region of the SARS-CoV-2 genome coding for the spike protein. The sequence of the S gene is described under GenBank (NCBI) accession number NC_045512.2: 21,563-25,384. For purposes of the present invention, region S.45 comprises the nucleotide sequence defined as SEQ ID NO: 3: which is located between positions 21,665-21,885 of the SARS CoV-2 genome described under NC_045512.2. Said SEQ ID NO: 3 is represented by the following nucleotide sequence: 
ggtgtttattaccctgacaaagttttcagatcctcagttttacattcaactcaggacttgttcttacctttcttttcc
aatgttacttggttccatgctatacatgtctctgggaccaatggtactaagaggtttgataaccctgtcctac
catttaatgatggtgtttattttgcttccactgagaagtctaacataataagaggctggatttttggtac.
- Region ORF1ab.99: as it is herein used, said region can be found in the region of the SARS-CoV-2 genome of the *ORF1ab* gene. The sequence of said gene is described under GenBank (NCBI) accession number NC_045512.2: 266-21,555. For purposes of the present invention, region ORF1ab.99 comprises the nucleotide sequence defined as SEQ ID NO: 4 which is located between positions 18,704-18,904 of the SARS CoV-2 genome described under NC_045512.2. Said SEQ ID NO: 4 is represented by the following nucleotide sequence: 
acacttatgcctgttggcatcattctattggatttgattacgtctataatccgtttatgattgatgttcaacaatgg
ggttttacaggtaacctacaaagcaaccatgatctgtattgtcaagtccatggtaatgcacatgtagctagt
tgtgatgcaatcatgactaggtgtctagctgtccacgagtgctttgttaagc.
- Region E.105: as it is herein used, it refers to the region coding for the envelope protein. The sequence of the E gene is described under GenBank (NCBI) accession number NC_045512.2: 26,000-26,600. For purposes of the present invention, region E.105 comprises the nucleotide sequence defined as SEQ ID NO: 5, which is located between positions 26,216-26,441 of the SARS CoV-2 genome described under NC_045512.2. Said SEQ ID NO: 5 is represented by the following nucleotide sequence:

tgtaagcacaagctgatgagtacgaacttatgtactcattcgtttcggaagagacaggtacgttaatagtt
aatagcgtacttctttttcttgctttcgtggtattcttgctagttacactagccatccttactgcgcttcgattgtgtg
cgtactgctgcaatattgttaacgtgagtcttgtaaaaccttctttttacgtttactctcgtgttaaaaatctgaa.

Next, the different sets of primers for the isothermal amplification system were designed using specific *PrimerExplorer v5* software (Eiken, Japan) and *LAMP Designer* software (Premier Biosoft). Different designs were proposed for each region of the SARS-CoV-2 virus genome mentioned above. Specifically, nine sets of LAMP primers were designed for region ND1-1 (SEQ ID NO: 1) which were located in different regions of said sequence. After the homology study, two of these nine sets were discarded, and seven sets of primers were selected for region ND1-1 (SEQ ID NO: 1), the results shown in the examples included in the present documents have been carried out with one of the seven sets designed. For the region spanning from the 5' end of the nucleoprotein coded by the N gene to the 3' end of the region of the ORF8 gene, region ND3B (SEQ ID NO: 2), two sets of LAMP primers have been designed, and the results shown in the examples included in the present documents have been carried out with one of the two sets designed. For region S.45 (SEQ ID NO: 3) four sets of LAMP primers have been designed, and two of them have been selected, although the results shown in the examples included in the present documents have been carried out for just one of the sets. For region ORF1ab.99 (SEQ ID NO: 4), fourteen sets of LAMP primers have been designed, and ten of them have been worked up experimentally, although the results shown in the examples included in the present documents have been carried out with one of the ten sets designed. Lastly, for region E.105 (SEQ ID NO: 5), five sets of LAMP primers have been designed, and three of them have been worked up experimentally, but the results shown in the examples included in the present documents have been carried out with one of the five sets designed.

Once the areas of the SARS-CoV-2 virus genome in which the primers were going to be designed were selected, sequences homology were made with the BioEdit v7.0.5.3 program against a virus with sequences with a high homology with SARS-CoV-2, such as MERS coronavirus or SARS-CoV-1 from 2003, for the purpose of checking that the areas selected for the design of said LAMP primers are completely specific to the SARS-CoV-2 virus. The results obtained clearly showed that all the primer designs of used in the present invention comprise genetic sequences specific to the SARS-CoV-2 virus.

In a preferred embodiment, the set of LAMP primers for the amplification of the specific regions of the SARS-CoV-2 virus described in the present invention comprises at least 6 primers for each of said mentioned regions.

**Table 1 shows the different sequences of the LAMP primer designed for amplifying the specific regions of the SARS-CoV-2 virus genome according to the present invention.**

| **Gene** | **Region** | **Name of the primer** | **Nucleotide sequence (SEQ ID NO: )** |
|---|---|---|---|
| *N* | **ND1-1** (SEQ ID NO: 1) | ND1-1 F3 | TGGACCCCAAAATCAGCG (SEQ ID NO: 6) |
| | | ND1-1 B3 | GCCTTGTCCTCGAGGGAAT (SEQ ID NO: 7) |
| | | ND1-1 FIP | CCACTGCGTTCTCCATTCTGGTAAATGCACCCCG CATTACG (SEQ ID NO: 8) |
| | | ND1-1 BIP | CGCGATCAAAACAACGTCGGCCCTTGCCATGTT GAGTGAGA (SEQ ID NO: 9) |
| | | ND1-1.22 LF | CAGTTGAATCTGAGGGTCCACCAAA (SEQ ID NO: 10) |
| | | ND1-1.22 LB | GTTTACCCAATAATACTGCGTCTTG (SEQ ID NO: 11) |
| *ORF8* | **ND3B** | ND3B-1 F3 | CCTTTTACAATTAATTGCCAGGA |
| *-N* | (SEQ ID NO: 2) | | (SEQ ID NO: 12) |
| | | ND3B-1 B3 | CCACTGCGTTCTCCATTC (SEQ ID NO: 13) |
| | | ND3B-1 FIP | ACACGAACGTCATGATACTCTAAAAACCTAAATT GGGTAGTCTTGT (SEQ ID NO: 14) |
| | | ND3B-1 BIP | GGACCCCAAAATCAGCGAAATTGCCAGTTGAATC TGAGG (SEQ IU NU: 15) |
| | | ND3B-1.3 LF | TTCATAGAACGAACAACGCACT (SEQ ID NO: 16) |
| | | ND3B-1.3 LB | GCACCCCGCATTACGTTTG (SEQ ID NO: 17) |
| *S* | **S.45** (SEQ ID NO: 3) | S.45 F3 | GGTGTTTATTACCCTGACAAAG (SEQ ID NO: 18) |
| | | S.45 B3 | GTACCAAAAATCCAGCCTC (SEQ ID NO: 19) |
| | | S.45 FIP | CATGGAACCAAGTAACATTGGAAAATTTTCAGAT CCTCAGTTTTACATTC (SEQ ID NO: 20) |
| | | S.45 BIP | CTCTGGGACCAATGGTACTAAGAGGACTTCTCAG TGGAAGCA (SEQ ID NO: 21) |
| | | S.45 LF | GAAAGGTAAGAACAAGTCCTGAGTT (SEQ ID NO: 22) |
| | | S.45 LB | GTTTGATAACCCTGTCCTACCATT (SEQ ID NO: 23) |
| *ORF1 ab* | **ORF1ab.99** (SEQ ID NO: 4) | ORF1ab.99 F3 | ACACTTATGCCTGTTGGC (SEQ ID NO: 24) |
| | | ORF1ab.99 B3 | GCTTAACAAAGCACTCGTG (SEQ ID NO: 25) |
| | | ORF1ab.99 FIP | GGTTGCTTTGTAGGTTACCTGTAAATTTGATTACG TCTATAATCCGTT (SEQ ID NO: 26) |
| | | ORF1ab.99 BIP | GATCTGTATTGTCAAGTCCATGGTGACAGCTAGA CACCTAGTCAT (SEQ ID NO: 27) |
| | | ORF1ab.99 LF | CCCCATTGTTGAACATCAATCA (SEQ ID NO: 28) |
| | | ORF1ab.99 LB | AATGCACATGTAGCTAGTTGTG (SEQ ID NO: 29) |
| *E* | **E.105** (SEQ ID NO: 5) | E.105 F3 | TGTAAGCACAAGCTGATGA (SEQ ID NO: 30) |
| | | E.105 B3 | TTCAGATTTTTAACACGAGAGT (SEQ ID NO: 31) |
| | | E.105 FIP | CGAAAGCAAGAAAAAGAAGTACGCTCGAACTTAT GTACTCATTCGTTTC (SEQ ID NO: 32) |
| | | E.105 BIP | TTGCTAGTTACACTAGCCATCCTTAGGTTTTACAA GACTCACGT (SEQ ID NO: 33) |
| | | E.105 LF | ACTATTAACGTACCTGTCTCTTCC (SEQ ID NO: 34) |
| | | E.105 LB | ACTGCGCTTCGATTGTGTG (SEQ ID NO: 35) |

Thus, in a preferred embodiment, step b) of the first method of the invention comprises the isothermal amplification RT-LAMP of at least two specific regions of the SARS-CoV-2 virus genome which are selected from region ND3B of SEQ ID NO: 2 and region S.45 of SEQ ID NO: 3.

In another more preferred embodiment, the set of LAMP primers for amplifying the region ND3B of SEQ ID NO: 2 comprises a set of six primers, which are the following:
- a forward primer F3 comprising a nucleotide sequence of SEQ ID NO: 12,
- a backward primer B3 comprising a nucleotide sequence of SEQ ID NO: 13,
- a forward inner primer FIP comprising a nucleotide sequence of SEQ ID NO: 14,
- a backward inner primer BIP comprising a nucleotide sequence of SEQ ID NO: 15,
- a forward loop primer LF comprising a nucleotide sequence of SEQ ID NO: 16; and
- a backward loop primer LB comprising a nucleotide sequence of SEQ ID NO: 17.

In another more preferred embodiment, the set of LAMP primers for amplifying the region S.45 of SEQ ID NO: 3, comprises a set of six primers, which are the following:
- a forward primer F3 comprising a nucleotide sequence of SEQ ID NO: 18,
- a backward primer B3 comprising a nucleotide sequence of SEQ ID NO: 19,
- a forward inner primer FIP comprising a nucleotide sequence of SEQ ID NO. 20,
- a backward inner primer BIP comprising a nucleotide sequence of SEQ ID NO: 21,
- a forward loop primer LF comprising a nucleotide sequence of SEQ ID NO: 22, and
- a backward loop primer LB comprising a nucleotide sequence of SEQ ID NO: 23.

In another preferred embodiment of the first method of the invention, the amplification solution or mixture comprises at least the sets of primers aforementioned. Said sets of primers can be found in the amplification mixture in a concentration ranging between the following concentrations based on the type of primer:
- FIP and BIP primers can be found in the amplification mixture in a range of concentrations between 1,000 nM and 2,000 nM, preferably between 1,200 and 1,800 nM, more preferably between 1,400 and 1,600 nM, even more preferably, the concentration of the FIP and BIP primer in the amplification mixture is 1,600 nM.
- LB and LF primers can be found in the amplification mixture in a range of concentrations between 400 nM and 1,400 nM, preferably between 600 and 1,200 nM, more preferably between 800 and 1,000 nM, even more preferably, the concentration of the LB and LF primer in the amplification mixture is 800 nM.
- F3 and B3 primers can be found in the amplification mixture in a range of concentrations between 80 nM and 600 nM, preferably between 100 and 400 nM, more preferably between 150 and 250 nM, even more preferably, the concentration of the F3 and B3 primers in the amplification mixture is 200 nM.

In a preferred embodiment of step b) of the first method of the invention, the amplification solution, in addition to comprising the set of LAMP primers designed for amplifying specific regions of the SARS-CoV-2 virus genome mentioned earlier, additionally comprises at least one isothermal DNA polymerase, at least one cofactor, a mixture of deoxyribonucleotide triphosphate (dNTP), and optionally, at least one DNA stabiliser.

Suitable DNA polymerases according to the invention are those which demonstrate a robust yield and have a significantly high reverse transcriptase activity. In a preferred embodiment, the DNA polymerase is a DNA polymerase BST, preferably *Bst 2.0* or *Bst large,* more preferably the DNA polymerase enzyme *Bst large.*

In a preferred embodiment, the DNA polymerase is comprised in the amplification solution in a range between 5 and 20 units. Preferably, the DNA polymerase is comprised in a range between 10 and 20 units, more preferably from 12 to 18 units, more preferably from 14 to 16 units, and even more preferably, the DNA polymerase is comprised in the amplification solution at a concentration of 12 units.

According to a preferred embodiment of the invention, the at least one cofactor is selected from the group consisting of: MgSO₄, 2-(trimethylazonium) acetate, or mixtures thereof. In a more preferred embodiment, the cofactor is MgSO₄. In another preferred embodiment, the at least one cofactor is comprised in a concentration between 1 mM and 10 mM. Preferably, the at least one cofactor is comprised in a concentration between 2 mM and 8 mM, more preferably between 4 and 8 mM, more preferably between 4 and 6 mM and even more preferably, the concentration of the at least one cofactor in the amplification solution is 6 mM.

In another preferred embodiment, the mixture of dNTPs comprised in the amplification solution is in a range of concentrations between 1.0 and 2.5 mM each, preferably between 1.2 and 2 mM each, more preferably between 1.4 and 1.6 mM, and even more preferably, the mixture of dNTPs comprised in the amplification solution is at a concentration of 1.4 mM each.

According to a preferred embodiment of the invention, the at least one DNA stabiliser, which may or may not be present in the amplification solution, when present, is chosen from the group consisting of glycerol, polyethylene glycol, formamide, betaine, 7-deaza-dGTP, dimethylsulfoxide and dITP. In a more preferred embodiment, the DNA stabiliser is betaine. In another embodiment of the invention, when the at least one DNA stabiliser is comprised in the amplification solution, it is at a concentration between 0.1 and 1 M, more preferably between 0.2 and 0.8 M, more preferably between 0.4 and 0.6 M and even more preferably at a concentration of 0.6 M.

In another preferred embodiment, as mentioned earlier, the amplification solution is comprised in the reading reagent of the present invention. Thus, for purposes of the present invention, the term "reading reagent" refers to the amplification solution of the present invention, additionally comprising a reagent which allows the detection of the presence of the amplified regions by means of an optical detection, preferably by means of fluorescence detection, colorimetric detection and/or chemiluminescent detection. In a more preferred embodiment, the detection is carried out by means of colorimetric or fluorescent methods. Thus, in a more preferred embodiment, when optical detection is carried out, the reading reagent of the present invention is selected from any compound that emits fluorescence and/or changes colour during the LAMP amplification process, such as for example, Syber Green, hydroxynaphthol blue, neutral red, crystal violet, calcein, Evagreen and SYTO 9. In an even more preferred embodiment, the reading reagent is selected from calcein and Evagreen.

Throughout LAMP isothermal amplification of the genetic material, the reaction produces high amounts of pyrophosphate as a by-product of the enzymatic reaction. This pyrophosphate reacts with the magnesium ions (cofactor) present in the amplification solution. These complexes can react with specific fluorophores, which can develop a change in colour in the reaction mixture that can be detected in the visible spectrum at the same time the reaction occurs. Thus, a direct reading of the reaction with the naked eye is possible.

In another preferred embodiment of the first method of the present invention, the amplification solution comprises a modulated frequency spectrum fluorophore (FAM). Suitable FAM fluorophores according to the invention comprise, but are not limited to: calcein, hydroxynaphthol blue (HNB), 6-carboxyfluorescein, Alexa fluor 488, FITC fluorescein, Syber Green, EvaGreen and SYTO 9. In a preferred embodiment of the invention, fluorophores from the FAM spectrum are preferably calcein and EvaGreen.

Thus, if the amplification solution comprises calcein, the presence of said fluorophore allows the colorimetric optical detection of the amplification product with the naked eye since the pyrophosphate generated during the LAMP amplification reaction precipitates in the presence of said calcein, and the colour turns from orange to dark yellow, which is detectable with the naked eye and which, therefore, allows ensuring whether or not the SARS-CoV-2 virus is present in the analysed sample.

Furthermore, if the reaction mixture comprises the fluorophore EvaGreen, said fluorophore emits fluorescence in the presence of the double strand of DNA generating an increasing emission of fluorescence that increases in intensity as the DNA is polymerised.

In another embodiment of the invention, when the modulated frequency spectrum fluorophore (FAM) is present in the amplification solution, it is at a concentration between 0.1 µM and 2 µM, preferably between 0.1 µM and 0.5 µM, preferably between 0.2 µM and 0.4 µM, even more preferably at a concentration of 0.2 µM

As shown in **Example 4** of the present invention, the optical detection method described in the present invention is capable of detecting 83 viral copies per reaction analysed for the assayed clinical sample, exceeding the limit of detection reached by Lalli, *et al.* (Lalli MA, et al. Clin Chem. 2021;67(2):415-24)of 100 viral copies per reaction by the colorimetric LAMP method marketed by New England Biolabs (WarmStart Colorimetric LAMP 2X Master Mix). The comparative assays for the specificity study shown in the present document (see **Example 4,** **Figure 21****)** have demonstrated that the commercial kit by New England Biolabs (WarmStart Colorimetric LAMP 2X Master Mix) detects RNA from SARS-CoV-1, SARS 2003, which is considered a false positive result, since it detects other types of viruses other than SARS-CoV-2. Furthermore, the results obtained with the commercial kit (SARS-CoV-2 Rapid colorimetric LAMP Assay Kit) and shown in **Example 4,** **Figure 22****,** have demonstrated false negative results, in other words, they did not detect the presence of the viral RNA in the sample previously doped with 500 RNA copies/mL of SARS-CoV-2, which, therefore, should be detected as positive for SARS CoV-2.

Thus, in another more preferred embodiment of the first method of the present invention, the amplification reagent for optical detection, the reading reagent for optical detection comprises an amplification solution comprising at least one isothermal polymerase, preferably polymerase *Bst large;* at least one cofactor, preferably MgSO₄; a mixture of deoxyribonucleotide triphosphate (dNTP); the set of LAMP primers described in the present invention, and at least one reading reagent comprising a modulated frequency spectrum fluorophore (FAM), and optionally, at least one DNA stabiliser, preferably betaine. In a more preferred embodiment, the reading reagent comprises an isothermal polymerase, preferably polymerase *Bst large;* in a range of 5 to 20 units; at least one cofactor, preferably MgSO₄ in a range of 1 mM to 10 mM; a mixture of dNTP in a concentration between 1.0 and 2.5 mM; the set of LAMP primers described in the present invention in a mean concentration between 80 nM and 6500 nM, at least one reading reagent comprising a modulated frequency spectrum fluorophore (FAM), preferably calcein or EvaGreen, in a concentration of 0.1 µM to 2 µM; and at least one DNA stabiliser, preferably betaine in a range of 0.1 to 1 M.

In another preferred embodiment, if the detection of step c) of the first method of the present invention is carried out by means of electrochemical methods, then the reading reagent of the present invention comprises the amplification solution as described above, but instead of comprising at least one reading reagent comprising a fluorophore FAM, said reading reagent comprises a redox compound. For purposes of the present invention, the redox compound is a substrate which is intercalated between the bases of the double strand of DNA which gradually reduces the difference in potential in the amplification solution, detectable by means of cyclic voltammetry, as the amplified product is generated, so it can be monitored in real time. In a more preferred embodiment, the reading reagent for electrochemical detection is selected from the list consisting of: methylene blue, toluidine blue, ferrocene, anthraquinone, osmium complexes, meldola blue, anthraquinone acids, daunomycin, Hoeschst 33258 and [Co(phen)₃](ClO₄)₃. In an even more preferred embodiment, the reading reagent is methylene blue.

Initially, the reading reagent for the electrochemical detection, preferably methylene blue, the methylene blue molecules are diffused freely in the amplification solution, and when they are reduced/oxidised, they transfer electrons to the electrode, generating a measurable electric current. During the LAMP reaction, the methylene blue molecule is intercalated in the amplicons of the double strand of DNA generated and its electron transfer reaction is hindered. Therefore, the reduction of free methylene blue results in a decrease of the electrochemical signal. This approach significantly reduces the electrochemical signal for differentiating between the presence of SARS-CoV-2 DNA (low electrochemical signal) and the absence of SARS-CoV-2 DNA (high electrochemical signal).

In comparison with optical detection, electrochemical detection has the advantage of showing high sensitivity and selectivity, a high signal/noise ratio, simple equipment, a low cost and very rapid response time, which perfectly fits with the strong points of the LAMP reaction for *in situ* determinations of the presence of SARS-CoV-2 in a sample from a subject. Therefore, this method provides a direct way to distinguish the changes in pH that are really due to specific amplifications and are not false positives.

In a preferred embodiment, the redox compound for electrochemical detection is comprised in the reading reagent in a concentration between 1 µg/ml and 1000 µg/mL, preferably between 30 µg/mL and 700 µg/mL, preferably between 50 µg/mL and 500 µg/mL, preferably between 100 µg/ml and 300 µg/mL, more preferably at a concentration of 200 µg/mL.

Thus, in a more preferred embodiment of the first method of the present invention, the amplification reagent for electrochemical detection, the reading reagent for electrochemical detection comprises an amplification solution comprising at least one isothermal polymerase, preferably polymerase *Bst large;* at least one cofactor, preferably MgSO₄; a mixture of deoxyribonucleotide triphosphate (dNTP); the set of LAMP primers described in the present invention, and at least one reading reagent comprising a redox substrate or indicator, preferably methylene blue, and optionally, at least one DNA stabiliser, preferably betaine. In a more preferred embodiment, said detection reagent comprises an isothermal polymerase, preferably polymerase *Bst large;* in a range of 5 to 20 units; at least one cofactor, preferably MgSO₄ in a range of 1 mM to 10 mM; a mixture of dNTP in a concentration between 1.0 and 2.5 mM; the set of LAMP primers described in the present invention in a mean concentration between 80 nM and 6500 nM, at least one reading reagent comprising a redox substrate or indicator, preferably methylene blue, in a concentration between 1 µg and 1,000 µg/mL; and at least one DNA stabiliser, preferably betaine in a range of 0.1 to 1 M.

In another preferred embodiment, the LAMP isothermal amplification reaction of step b) of the first method of the invention is carried out at a temperature between 60 and 70 °C, preferably between 62 °C and 65 °C, more preferably the LAMP amplification reaction was carried out at a temperature of 63 °C.

In another preferred embodiment of the first method of the invention, in step c) the reading and detection of the presence or absence of at least one amplification product of the specific regions of step b) is performed by means of an optical and/or electrochemical method. As was previously mentioned, the presence of specific fluorophores in the amplification reagent allows the direct reading of the reaction with the naked eye:
a) in the visible spectrum by means of the colorimetric change due to precipitation of the pyrophosphate generated during the amplification reaction in the presence of a substrate, such as calcein, causing the colour to change from orange to dark yellow over the course of the amplification reaction, and
b) in the fluorescent spectrum when excitation of the solution occurs at a wavelength of 496 nm, an increase in fluorescence emission is generated in the green fluorescent spectrum, which increases in intensity as the DNA is polymerised.

Furthermore, the presence of the redox indicator in the amplification sample allows the detection of the amplified products in real time and in the visible spectrum by means of electrochemical methods known to any person skilled in the art. The electrochemical determinations in the present invention are performed by cyclic voltammetry.

In order to evaluate whether the LAMP reaction has occurred, therefore indicating the presence of the specific target sequence of COVID-19, the height of the oxidation peak corresponding to the redox substrate is compared; in the examples of the present invention, methylene blue (control) has been used. When amplification occurs in a satisfactory manner, the methylene blue is intercalated in the new DNA copies generated, thus limiting their susceptibility to experiencing oxidation/reduction, culminating with a detectable decrease in electron transfer. This lower current intensity translates into peaks having a smaller amplitude in cyclic voltammetry. **Figure 2** shows a diagram of the detection method for the detection of SARS-CoV-2 by means of LAMP isothermal amplification and electrochemical detection.

In a preferred embodiment of the first method of the present invention, said method is characterised in that step c) is preferably carried out by means of an electrochemical method.

In a second aspect, the present invention relates to an *in vitro*/*ex vivo* method for the diagnosis of the presence of SARS-CoV-2 in an isolated biological sample, wherein said isolated biological sample preferably belongs to a subject. The second method of the present invention comprises the same steps a) to c) as those described above for the first method of the invention; therefore, all the information mentioned throughout the present document for the first method of the invention applies *mutatis mutandis* to the second method of the invention, with the particularity that the second method of the invention comprises an additional step in which after detection of the presence or absence of the amplification product of the specific regions of the SARS-CoV-2 virus in step c), if:
i. the presence of amplification product for the two specific regions of SEQ ID NOs: 2 and 3 is detected, as mentioned in step b), said detection is indicative of the presence of SARS-CoV-2 in the sample, and, therefore, the subject suffers from COVID-19, and
ii. if the absence of amplification product for the two specific regions of SEQ ID NOs: 2 and 3 is detected, as mentioned in step b), said absence of detection is indicative of the absence of SARS-CoV-2 in the sample, and, therefore, the subject does not suffer from COVID-19.

In a preferred embodiment both of the first method of the invention and of the second method of the invention, if the presence of amplification product for one of the regions of SEQ ID NOs: 2 or 3 is detected in step c), and the presence of amplification product for the other region of SEQ ID NOs: 2 or 3 is not detected, respectively, the result would be considered questionable, and step b) of LAMP isothermal amplification would have to be carried out again, but specific regions of SARS-CoV-2 other than the regions of SEQ ID NOs: 2 and 3 will be amplified, and said new regions are selected from the list consisting of region ND1-1 (SEQ ID NO: 1), region ORF1ab.99 (SEQ ID NO: 4) and/or region E.105 (SEQ ID NO: 5). Thus, when one of SEQ ID NOs: 2 or 3 gives rise to an amplification product and the other one does not, steps b) and c) of the two methods of the invention will be carried out again in the genetic material extracted from the isolated sample, but now the amplification solution will comprise the set of LAMP primers specific for each of the new regions of SEQ ID NOs. 1, 4 and/or 5, i.e., the primers specific for the amplification of the regions of SEQ ID NO: 1, SEQ ID NO: 4 and/or SEQ ID NO: 5, which are described in **Table 1.** Thus, the sample will be assayed for the regions of SEQ ID NO: 1, SEQ ID NO: 4 and SEQ ID NO: 5, successively until at least one of said regions gives rise to an amplification product, and then said sample can be classified as positive for the presence of SARS-CoV-2, or COVID-19.

As such, in order to have a definitive final result of the presence of SARS-CoV-2 in the analysed sample, at least two of the regions of the SARS-CoV-2 genome analysed must give rise to amplification products in step b) which are detected in step c) of the methods of the present invention.

In a preferred embodiment, the sets of primers for the amplification of the regions of SEQ ID NO: 1, SEQ ID NO: 4 and/or SEQ ID NO: 5 of the SARS-CoV-2 virus used in the methods of the present invention are the following:
- *Set of LAMP Primers for amplifying region* ND1-1 of *SEQ ID NO: 1:*
   - a forward primer F3 comprising a nucleotide sequence of SEQ ID NO: 6,
   - a backward primer B3 comprising a nucleotide sequence of SEQ ID NO: 7,
   - a forward inner primer FIP comprising a nucleotide sequence of SEQ ID NO: 8,
   - a backward inner primer BIP comprising a nucleotide sequence of SEQ ID NO: 9,
   - a forward loop primer LF comprising a nucleotide sequence of SEQ ID NO: 10; and
   - a backward loop primer LB comprising a nucleotide sequence of SEQ ID NO: 11.
- *Set of LAMP Primers for amplifying region* ORF1ab.99 *(SEQ ID NO: 4):*
   - a forward primer F3 comprising a nucleotide sequence of SEQ ID NO: 24,
   - a backward primer B3 comprising a nucleotide sequence of SEQ ID NO: 25,
   - a forward inner primer FIP comprising a nucleotide sequence of SEQ ID NO. 26,
   - a backward inner primer BIP comprising a nucleotide sequence of SEQ ID NO: 27,
   - a forward loop primer LF comprising a nucleotide sequence of SEQ ID NO: 28, and
   - a backward loop primer LB comprising a nucleotide sequence of SEQ ID NO: 29.
- *Set of LAMP Primers for amplifying the region E105 (SEQ ID NO: 5):*
   - a forward primer F3 comprising a nucleotide sequence of SEQ ID NO: 30,
   - a backward primer B3 comprising a nucleotide sequence of SEQ ID NO: 31,
   - a forward inner primer FIP comprising a nucleotide sequence of SEQ ID NO. 32,
   - a backward inner primer BIP comprising a nucleotide sequence of SEQ ID NO: 33,
   - a forward loop primer LF comprising a nucleotide sequence of SEQ ID NO: 34, and
   - a backward loop primer LB comprising a nucleotide sequence of SEQ ID NO: 35.

In a third aspect, the present invention relates to the set or sets of LAMP primers, set or sets of primers of the present invention, for amplifying the specific regions of the SARS-CoV-2 virus described in the present invention, preferably for the amplification of the regions identified in the present invention as SEQ ID NOs: 1 to 5.

Thus, in a more preferred embodiment, the set of primers described in the present invention is shown in **Table 1,** as well as in the description of the methods of the present invention.

In a fourth aspect, the present invention relates to the use of the LAMP primers of the present invention for detecting the presence of the SARS-CoV-2 virus and/or for the diagnosis of the SARS-CoV-2 virus, in an isolated biological sample, preferably by means of the methods described in the present invention.

In a fifth aspect, the present invention relates to a reading reagent, hereinafter, reading reagent of the present invention, for detecting the presence of the amplification products of specific regions of the SARS-CoV-2 virus described in the present invention, and which are preferably the regions comprising SEQ ID NOs: 1 to 5, and wherein said detection reagent comprises at least at least one amplification solution as described above throughout the present document, and therefore it also comprises the set of LAMP primers of the present invention for the identification and amplification of the regions of the SARS-CoV-2 virus in the isolated biological sample analysed.

In a preferred embodiment of the reading reagent, said reading reagent is characterised in that when optical detection is carried out in step c) of the methods described in the present document, said reading reagent comprises at least one fluorophore, as described above.

In another preferred embodiment of the reading reagent, said reading reagent is characterised in that when electrochemical detection is carried out in step c) of the methods described in the present document, said reading reagent additionally comprises at least one indicator or redox substrate, as described above.

In a sixth aspect, the present invention relates to the use of the reading reagent of the present invention for detecting the presence of the SARS-CoV-2 virus and/or for the diagnosis of the SARS-CoV-2 virus, in an isolated biological sample from a subject, preferably by means of the methods described in the present invention.

In a seventh aspect, the present invention relates to a kit, device, biosensor or POC, hereinafter, kit, device, biosensor or POC of the invention, terms which will be used interchangeably throughout the present document, for the detection of SARS-CoV-2 in an isolated biological sample, wherein the said kit, device or POC comprises reagents for amplifying the genetic material in said sample using a LAMP technique, the set of LAMP primers as described in the present invention and/or the reading reagent as described in the present invention, preferably by means of the methods described in the present invention.

In a preferred embodiment, the kit, device or POC of the invention is adapted for the electrochemical detection of SARS-CoV-2, and the different reagents which said kit comprises are in turn adapted for the same purpose, as previously described herein.

The kits, devices or POCs for electrochemical detection are also called electrochemical biosensors in the state of the art. In comparison with optical biosensors, such as fluorescent, colorimetric and chemiluminescent assays, electrochemical biosensors have the advantages of high sensitivity and selectivity, a high signal/noise ratio, simple equipment, a low cost and rapid response time, which perfectly fits with the strong points of the LAMP reaction for *in situ* determinations. The electrochemical detection in this type of sensors is carried out, as mentioned earlier, by means of the detection of redox molecules in the presence or absence of amplification products.

Specifically, electrochemical detection can be performed on a sample previously amplified by means of LAMP and subsequently introduced in a disposable cartridge for electrochemical analysis, or both LAMP amplification and electrochemical detection can be performed in the same disposable cartridge. The electrochemical reading is performed by applying cyclic voltammetry by means of a portable analyser, and the presence or absence of the virus is confirmed by the magnitude of the redox molecule oxidation peak. In the present invention, the disposable cartridge together with the portable analyser constitute a POC. In the case in which amplification is performed in the actual cartridge, the POC would consist of a disposable cartridge, a portable analyser and a heating module.

In an eighth aspect, the present invention relates to the use of the kit, device, biosensor or POC of the present invention, for detecting the presence of the SARS-CoV-2 virus and/or for the diagnosis of the SARS-CoV-2 virus, in an isolated biological sample from a subject.

### EXAMPLES

Next, the invention will be illustrated by means of assays carried out by the inventors, which clearly show the effectiveness of the product of the invention. The following examples serve to illustrate the invention and must not be regarded as being limiting of the scope thereof.

### EXAMPLE 1. Optimisation of specificity conditions for optical detection of SARS-Cov-2 by means of the method of the present invention.

For the specificity studies when determining the presence or absence of SARS-CoV-2 in a sample by means of optical detection, SARS-CoV-2 virus RNA and RNA from exclusive viruses (other than SARS-CoV-2) **Table 2,** were used. Additionally, other related coronaviruses and a mixture of inactivated viruses which commonly affect the upper respiratory tract with symptoms very similar to those of COVID-19. All the assays were performed with AmpliRun RNA (Vircell). For the inactivated viruses which commonly affect the upper respiratory tract, viral RNA was extracted using Viral RNA Mini Kit (Qiagen). The objective of said assays is to experimentally confirm that the detection by means of the methods and primers described in the present invention is completely specific for SARS-CoV-2 when detected by means of optical methodology.

**Table 2. List of inclusive/exclusive viruses used for the homology and specificity study.**

| **Inclusive virus RNA** | **Exclusive virus RNA** | **Panel of exclusive respiratory viruses** |
|---|---|---|
| SARS-CoV-2 | - Coronavirus OC43 | - Adenovirus 4 |
| | - MERS coronavirus | - Coronavirus OC43 |
| | - Rhinovirus | - Influenza A H3N2 |
| | - Enterovirus 68 | - Influenza B, |
| | - SARS coronavirus 2003 | - Novel Influenza A H1N1 |
| | | - Parainfluenza 1 |
| | | - Parainfluenza 2 |
| | | - Parainfluenza 3 |
| | | - Respiratory syncytial virus, subtype A |
| | | - Respiratory syncytial virus, subtype B |

For the biochemical optimisation of each of the designs of the detection reagents, calcein has been used to monitor the change in colour occurring when the biological test sample contains SARS-CoV-2 viral RNA. Another fluorophore, EvaGreen, has also been used, and the way it works on a chemical level is more similar to the redox compound, methylene blue, used for the electrochemical reading, given its capacity to be intercalated in the DNA double helix as amplification of the SARS-CoV-2 DNA takes place.

All the sets of primers were tested to confirm that primer dimers are not formed, since the generation of dimers generates results which often cannot be distinguished from positive results, giving what is known as false positive results. Once it was confirmed that all the sets of primers functioned correctly (data not shown), the concentrations and conditions for the different detection reagents were determined based on the specific primers used for each of the regions of SARS-CoV-2 used in the present invention.

For the LAMP reaction, standard conditions were the following: initial temperature of 65 °C; primer FIP/BIP, 1.6 mM; primer LB/LF, 800 nM; primer F3/B3 200 nM; 1X buffer, MgSO₄, 6 mM; Betaine, 0.4 M; dNTPs, 1.4 mM and the enzyme *Bst large* was used. All the reactions were carried out in a time of 50 minutes.

For the optimisation of the different parameters of the RT-LAMP isothermal amplification reaction, the temperature gradient between 60 and 65 °C was established, concentrations of betaine between 0 and 0.6 M were tested, and assays were performed with concentrations of MgSO₄ between 4 and 8 mM. In order to decide what the optimal concentration was, the Cq in which the assayed samples were amplified was taken into account. The Cq is the time in which fluorescence emission is detectable, each Cq is equivalent to one minute of the reaction. Therefore, the lower the Cq, the higher the yield of the amplification is. Said process was the same for all the assayed detection reagents comprising the specific sets of LAMP primers amplifying the specific regions of the SARS-CoV-2 virus and defined as SEQ ID NO: 1 to 5 in the present invention. Shown next are two examples for the determination of the amplification conditions for region ND1-1 of SEQ ID NO: 1 and for region ND3B of SEQ ID NO: 2.

In the first optimisation example shown, region ND1-1 (SEQ ID NO: 1) was used, where the comparison of amplification efficiency with three different concentrations of MgSO₄ in the final volume of the reaction can be seen. The assays were carried out in triplicate. As can be seen in Table 3, the concentration of MgSO₄, 6 mM turned out to confer a higher efficiency to the reaction.

**Table 3. Table of results expressing the Cq value of all the measurements obtained for the MgSO₄ variable. NC is the negative control of the reaction that does not comprise viral RNA.**

| **Concentration of MgSO₄** | **Cq1** | **Cq2** | **Cq3** |
|---|---|---|---|
| 4 mM | 19.6 | 19.3 | 20.07 |
| **6 mM** | **13.7** | **13.7** | **13.5** |
| 8 mM | 16.25 | 16.11 | 16.03 |
| NC | NEG | NEG | NEG |

In the following optimisation example, region ND3B (SEQ ID NO: 2) was used, where the comparison of amplification efficiency with three different concentrations of betaine in the final volume of the reaction can be seen. The assays were carried out in triplicate. As can be seen in **Table 4,** the concentration of betaine, 0.2 M turned out to have the highest efficiency, but it presented a baseline detection with one of the negative controls. For this reason, use of a 0.4 M concentration of betaine was established for this region.

**Table 4. Table of results expressing the Cq value of all the measurements obtained for the betaine variable. NC is the negative control of the reaction that does not comprise viral RNA.**

| **Concentration of Betaine** | **Cq1** | **Cq2** | **Cq3** |
|---|---|---|---|
| 0.2 M | 7.28 | 7.71 | 7.19 |
| **0.4 M** | **8.88** | **8.56** | **8.55** |
| 0.6 M | 11.12 | 10.65 | 10.93 |
| NC | NEG | NEG | 33.5 |

Once the different assays were carried out with the different concentrations for each of the reagents making up the detection reagents of the present invention for each of the regions of SARS-CoV-2 detected in the present invention, the amplification conditions for each of the regions of SEQ ID NO: 1 to 5 analysed in the present document are described in detail below.
- Detection reagent of region ND1-1 of SEQ ID NO: 1 of SARS-CoV-2: MgSO₄, 6 mM; Betaine, 0.4 M; primers FIP (SEQ ID NO: 8) and BIP (SEQ ID NO: 9), 1,600 nM; primers LB (SEQ ID NO: 11) and LF (SEQ ID NO: 10), 800 nM; primers F3 (SEQ ID NO: 6) and B3 (SEQ ID NO: 7), 200 nM; dNTP mix, 1.4 mM (each nucleotide); *Bst large,* 12 u; Reverse transcriptase 20 u; and PCR-grade water to 25 µl of reaction. Reaction temperature/time: 63 °C/40 min.
- Detection reagent of region N D3B of SEQ ID NO: 2 of the SARS-CoV-2 virus: MgSO₄, 6 mM; Betaine, 0.4 M; primers FIP (SEQ ID NO: 14) and BIP (SEQ ID NO: 15), 1,600 nM; primers LB (SEQ ID NO: 17) and LF (SEQ ID NO: 16), 800 nM; primers F3 (SEQ ID NO: 12) and B3 (SEQ ID NO: 13), 200 nM; dNTP mix, 1.4 mM (each nucleotide); *Bst large,* 12 u; Reverse transcriptase 20 u; and PCR-grade water to 25 µl of reaction. Reaction temperature/time: 63 °C/40 min.
- Detection reagent of region S.45 of SEQ ID NO: 3 of the SARS-CoV-2 virus: MgSO₄, 6 mM; Betaine, 0.4 M; primers FIP (SEQ ID NO: 20) and BIP (SEQ ID NO: 21), 1,600 nM; primers LB (SEQ ID NO: 23) and LF (SEQ ID NO: 22), 800 nM; primers F3 (SEQ ID NO: 18) and B3 (SEQ ID NO: 19), 200 nM; dNTP mix, 1.4 mM (each nucleotide); *Bst large,* 12 u; Reverse transcriptase 20 u; and PCR-grade water to 25 µl of reaction. Reaction temperature/time: 63 °C/40 min.
- Detection reagent of region ORF1ab.99 of SEQ ID NO: 4 of the SARS-CoV-2 virus: MgSO₄, 6 mM; primers FIP (SEQ ID NO: 25) and BIP (SEQ ID NO: 26), 1,600 nM; primers LB (SEQ ID NO: 29) and LF (SEQ ID NO: 28), 800 nM; primers F3 (SEQ ID NO: 28) and B3 (SEQ ID NO: 29), 200 nM; dNTP mix, 1.4 mM (each nucleotide); *Bst large,* 12 u; Reverse transcriptase 20 u; and PCR-grade water to 25 µl of reaction. Reaction temperature/time: 63 °C/40 min.
- Detection reagent of region E. 105 of SEQ ID NO: 5 of the SARS-CoV-2 virus: MgSO₄, 6 mM; primers FIP (SEQ ID NO: 32) and BIP (SEQ ID NO: 33), 1,600 nM; primers LB (SEQ ID NO: 35) and LF (SEQ ID NO: 34), 800 nM; primers F3 (SEQ ID NO: 30) and B3 (SEQ ID NO: 31), 200 nM; dNTP mix, 1.4 mM (each nucleotide); *Bst large,* 12 u; Reverse transcriptase 20 u; and PCR-grade water to 25 µl of reaction. Reaction temperature/time: 63 °C/40 min.

Once the LAMP isothermal amplification conditions were obtained, as well as the concentrations and components of the detection reagents, assays were carried out to determine the specificity of the method, primers and reagents of the present invention in the detection of SARS-CoV-2 in RNA samples of the virus in **Table 2.**

Next are shown the optical detection results, in the fluorescent spectrum: monitored in real time with the intercalating fluorophore EvaGreen, and at an end time, in the visible spectrum, assayed with calcein.

**Table 5** shows the results of the amplification assays performed in triplicate, monitored in real time in the reading with the FAM filter, and **Figure 3** shows the results in the visible spectrum for region ND1-1 of SEQ ID NO: 1 of the SARS-CoV-2 virus. As can be observed in said **Table 5,** the results are those expected by means of the two optical detection systems, the method used in the present invention is specific for the detection of SARS-CoV-2, and it does not detect any of the other assayed viruses.

**Table 5. Results obtained from the specificity assays performed in triplicate with 1,000 RNA copies of the inclusive and exclusive viruses in Table 2 for region ND1-1 of SEQ ID NO: 1 comprising the set of LAMP primers of SEQ ID NOs: 6 to 11. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **13.17** | **13.03** | **12.79** |
| Coronavirus OC43 | NEG | NEG | NEG |
| Enterovirus 68 | NEG | NEG | NEG |
| MERS Coronavirus | NEG | NEG | NEG |
| Rhinovirus | NEG | NEG | NEG |
| Coronavirus SARS 2003 | NEG | NEG | NEG |
| NC | NEG | NEG | NEG |

For assays performed with the panel of respiratory viruses, the extraction of the RNA of the Amplirun total respiratory viral panel control (swab) (Vircell), which is a panel with the mixture of 10 inactivated respiratory viruses (shown in **Table 2)** and formulations in viral transport medium, was performed. **Table 6** shows the results obtained in triplicate in real time in the reading with the FAM filter for region ND1-1 (SEQ ID NO: 1), and **Figure 4** shows the results in the visible spectrum for the same fragment. As can be seen the results are those expected by means of the two optical detection systems, the method used in the present invention is specific for the detection of SARS-CoV-2, and it does not detect any of the other assayed viruses.

**Table 6. Results obtained from the specificity assays performed in triplicate with RNA copies of SARS-CoV-2 and the mix of exclusive respiratory viruses in Table 2 for region ND1-1 of SEQ ID NO: 1 comprising the set of LAMP primers of SEQ ID NOs: 6 to 11. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **16.72** | **18.02** | **18.01** |
| Respiratory viruses | NEG | NEG | NEG |
| NC | NEG | NEG | NEG |

**Table 7** shows the results of the amplification assays performed in triplicate, monitored in real time in the reading with the FAM filter, and **Figure 5** shows the results in the visible spectrum for region ND3B of SEQ ID NO: 2 of the SARS-CoV-2 virus. As can be observed in said **Table 7,** the results are those expected by means of the two optical detection systems, the method used in the present invention is specific for the detection of SARS-CoV-2, and it does not detect any of the other assayed viruses.

**Table 7. Results obtained from the specificity assays performed in triplicate with 1,000 RNA copies of the inclusive and exclusive viruses in Table 2 for region ND3B of SEQ ID NO: 2 comprising the set of LAMP primers of SEQ ID NOs: 12 to 17. The Cq value of all the measurements obtained in the specificity assay is expressed. NC is the negative control of the reaction that does not comprise viral RNA. NEG: negative**

| **VIRAL RNA** | **Cq1** | **Cq2** | **Cq3** |
|---|---|---|---|
| **SARS-CoV2** | **11.28** | **11.17** | **11.13** |
| Coronavirus OC43 | NEG | NEG | NEG |
| Enterovirus 68 | NEG | NEG | NEG |
| MERS Coronavirus | NEG | NEG | NEG |
| Rhinovirus | NEG | NEG | NEG |
| Coronavirus SARS 2003 | NEG | NEG | NEG |
| NC | NEG | NEG | NEG |

For the assays performed with the panel of respiratory viruses for region ND3B (SEQ ID NO: 2), the same procedure as that mentioned above was followed. **Table 8** shows the results obtained in triplicate in real time in the reading with the FAM filter for region ND3B (SEQ ID NO: 2), and **Figure 6** shows the results in the visible spectrum for the same fragment. As can be seen the results are those expected by means of the two optical detection systems, the method used in the present invention is specific for the detection of SARS-CoV-2, and it does not detect any of the other assayed viruses.

**Table 8. Results obtained from the specificity assays performed in triplicate with RNA copies of SARS-CoV-2 and the mix of exclusive respiratory viruses in Table 2 for region ND3B of SEQ ID NO: 2 comprising the set of LAMP primers of SEQ ID NOs: 12 to 17. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **15.82** | **16.02** | **14.07** |
| Respiratory viruses | NEG | NEG | NEG |
| NC | NEG | NEG | NEG |

**Table 9** shows the results of the amplification assays performed in triplicate, monitored in real time in the reading with the FAM filter, and **Figure 7** shows the results in the visible spectrum for region S45 of SEQ ID NO: 3 of the SARS-CoV-2 virus. As can be observed in said **Table 9,** the results are those expected by means of the two optical detection systems, the method used in the present invention is specific for the detection of SARS-CoV-2, and it does not detect any of the other assayed viruses.

**Table 9. Results obtained from the specificity assays performed in triplicate with 1,000 RNA copies of the inclusive and exclusive viruses in Table 2 for region S45 of SEQ ID NO: 3 comprising the set of LAMP primers of SEQ ID NOs: 18 to 23. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **13.59** | **14.65** | **13.57** |
| Coronavirus OC43 | NEG | NEG | NEG |
| Enterovirus 68 | NEG | NEG | NEG |
| MERS Coronavirus | NEG | NEG | NEG |
| Rhinovirus | NEG | NEG | NEG |
| Coronavirus SARS 2003 | NEG | NEG | NEG |
| NC | NEG | NEG | NEG |

For the assays performed with the panel of respiratory viruses for region S45 (SEQ ID NO: 3), the same procedure as that mentioned above was followed. **Table 10** shows the results obtained in triplicate in real time in the reading with the FAM filter for region S45 (SEQ ID NO: 3), and **Figure 8** shows the results in the visible spectrum for the same fragment. As can be seen the results are those expected by means of the two optical detection systems, the method used in the present invention is specific for the detection of SARS-CoV-2, and it does not detect any of the other assayed viruses.

**Table 10. Results obtained from the specificity assays performed in triplicate with RNA copies of SARS-CoV-2 and the mix of exclusive respiratory viruses in Table 2 for region S45 of SEQ ID NO: 3 comprising the set of LAMP primers of SEQ ID NOs: 18 to 23. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **11.46** | **11.92** | **12.66** |
| Respiratory viruses | NEG | NEG | NEG |
| NC | NEG | NEG | NEG |

**Table 11** shows the results of the amplification assays performed in triplicate, monitored in real time in the reading with the FAM filter, and **Figure 9** shows the results in the visible spectrum for region ORF1ab.99 of SEQ ID NO: 4 of the SARS-CoV-2 virus. As can be observed in said **Table 11,** the results are those expected by means of the two optical detection systems, the method used in the present invention is specific for the detection of SARS-CoV-2, and it does not detect any of the other assayed viruses.

**Table 11. Results obtained from the specificity assays performed in triplicate with 1,000 RNA copies of the inclusive and exclusive viruses in Table 2 for region ORF1ab.99 of SEQ ID NO: 4 comprising the set of LAMP primers of SEQ ID NOs: 24 to 29. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **11.16** | **11.68** | **11.72** |
| Coronavirus OC43 | NEG | NEG | NEG |
| Enterovirus 68 | NEG | NEG | NEG |
| MERS Coronavirus | NEG | NEG | NEG |
| Rhinovirus | NEG | NEG | NEG |
| Coronavirus SARS 2003 | NEG | NEG | NEG |
| NC | NEG | NEG | NEG |

For the assays performed with the panel of respiratory viruses for region ORF1ab.99 (SEQ ID NO: 4), the same procedure as that mentioned above was followed. **Table 12** shows the results obtained in triplicate in real time in the reading with the FAM filter for region ORF1ab.99 (SEQ ID NO: 4), and **Figure 10** shows the results in the visible spectrum for the same fragment. As can be seen the results are those expected by means of the two optical detection systems, the method used in the present invention is specific for the detection of SARS-CoV-2, and it does not detect any of the other assayed viruses.

**Table 12. Results obtained from the specificity assays performed in triplicate with RNA copies of SARS-CoV-2 and the mix of exclusive respiratory viruses in Table 2 for region ORF1ab.99 of SEQ ID NO: 4 comprising the set of LAMP primers of SEQ ID NOs: 24 to 29. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **11.00** | **12.56** | **13.12** |
| Respiratory viruses | NEG | NEG | NEG |
| NC | NEG | NEG | NEG |

**Table 13** shows the results of the amplification assays performed in triplicate, monitored in real time in the reading with the FAM filter, and **Figure 11** shows the results in the visible spectrum for region E.105 of SEQ ID NO: 5 of the SARS-CoV-2 virus. As can be observed in said **Table 13,** the results are those expected by means of the two optical detection systems, the method used in the present invention is specific for the detection of SARS-CoV-2, and it does not detect any of the other assayed viruses.

**Table 13. Results obtained from the specificity assays performed in triplicate with 1, 000 RNA copies of the inclusive and exclusive viruses in Table 2 for region E. 105 of SEQ ID NO: 4 comprising the set of LAMP primers of SEQ ID NOs: 30 to 35. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **12.74** | **13.17** | **12.08** |
| Coronavirus OC43 | NEG | NEG | NEG |
| Enterovirus 68 | NEG | NEG | NEG |
| MERS Coronavirus | NEG | NEG | NEG |
| Rhinovirus | NEG | NEG | NEG |
| Coronavirus SARS 2003 | NEG | NEG | NEG |
| NC | NEG | NEG | NEG |

For the assays performed with the panel of respiratory viruses for region E.105 (SEQ ID NO: 5), the same procedure as that mentioned above was followed. **Table 14** shows the results obtained in triplicate in real time in the reading with the FAM filter for region E.105 (SEQ ID NO: 5), and **Figure 12** shows the results in the visible spectrum for the same fragment. As can be seen the results are those expected by means of the two optical detection systems, the method used in the present invention is specific for the detection of SARS-CoV-2, and it does not detect any of the other assayed viruses.

**Table 14. Results obtained from the specificity assays performed in triplicate with RNA copies of SARS-CoV-2 and the mix of exclusive respiratory viruses in Table 2 for region E. 105 of SEQ ID NO: 5 comprising the set of LAMP primers of SEQ ID NOs: 30 to 35. The Cq value of all the measurements obtained in the specificity assay is expressed. NC is the negative control of the reaction that does not comprise viral RNA. NEG: negative.**

| **VIRAL RNA** | **Cq1** | **Cq2** | **Cq3** |
|---|---|---|---|
| **SARS-CoV2** | **11.71** | **13.89** | **14.93** |
| Respiratory viruses | NEG | NEG | NEG |
| NC | NEG | NEG | NEG |

### EXAMPLE 2. Optimisation of specificity conditions for electrochemical detection of SARS-Cov-2 by means of the method of the present invention.

For the optimisation and validation of the method of the present invention when the detection of SARS-CoV-2 is carried out by electrochemical means, three repetitions for two samples have been used. The first sample is a commercial sample of SARS-CoV-2 inactivated viral particles comprising 1000 total copies (Amplirun^{®} Total SARS-CoV-2 Control, by Vircell), and the second sample is a negative control comprising exclusively water. The sets of assayed primers are the primers of SEQ ID NOs: 18 to 23, which amplify region S.45 of SEQ ID NO: 3, and in addition to the mentioned LAMP primers, the reading reagent comprises, instead of fluorophores, the redox substrate, which is methylene blue in the present example.

The electrochemical transduction method of the present invention has initially been validated in droplet form. In this format, the necessary volume of liquid is created by placing a droplet of the sample on the electrochemical biosensor placed horizontally. The method of RT-LAMP isothermal amplification is carried out with very small sample volumes (25 µl), which impedes working directly on commercial screen-printed carbon electrodes: the diameter of the semi-sphere formed by the droplet when it is placed on the sensing area does not entirely cover the three electrodes. Taking this into account, the inventors have designed a biosensor or kit on custom-made commercial electrodes having a working electrode diameter of 2 mm. The electrochemical determinations have been performed by cyclic voltammetry, in a potential window between -0.5 and 0.1 V with respect to a silver pseudo-reference electrode. The specific cyclic voltammetry parameters are included in **Table 15.**

**Table 15. Cyclic Voltammetry Parameters**

| Parameter | Value |
|---|---|
| E_step | 2 mV |
| S_rate | 100 mV/s |
| N_scans | 3 |
| E_begin | -0.5 V |
| E_1 | -0.5 V |
| E_2 | 0.1 V |

The biosensor or kit designed in the present invention has been carried out on glass and low-cost and single-use commercial electrochemical chips to prevent contaminations. The object of these biosensors is to have simple prototypes, useful as POC devices, which are manufactured rapidly with sufficient design flexibility to test different development and adapt them to the design requirements, and to perform the tests needed to demonstrate the usefulness thereof in the method of the present invention.

To manufacture the cells where the samples will be loaded for analysis, the following materials have been used on standard glass slides and on commercial electrochemical chips obtained from Meterhm Dropsens. The first material, referred to as Film 1 in the present invention, is ARsealTM90880 by Adhesive Research. It is a transparent polypropylene coated on both faces with PSA (pressure sensitive adhesive) and is received protected on both faces with PET (polyethylene terephthalate). Without the protective films, the material has a thickness of 142 µm. This thickness defines the height of the final chamber. This material has been chosen because of its evaporation barrier, because it is compatible with PCR processes and their reagents, has low autofluorescence and withstands rapid thermal cycles. It is furthermore resistant to DMSO solutions, acids, alcohols and organic solvents.

The following material referred to as Film 2 in the disposable cell prototype of the present invention is polycarbonate (PC), and commercial PC by Teckra with a thickness of 175 µm has been used.

The microfluidic connectors used in the prototype of the invention are polypropylene (PP) input connectors provided by ChipShop and plugs for the PP connectors provided by ChipShop. These plugs are used to prevent evaporation of the sample during heating and cross-contaminations. Two-sided tape with high heat resistance is used for adhering the connectors to the PC sheet (Film 2).

The cell and the channels, as well as the inlet and outlet openings are designed by means of the software incorporated in the cutting printer used. The designs are replicated in the different materials using a Silhouette Cameo^{®} cutting printer. By means of a small blade, this printer is capable of cutting the different proposed designs in the different materials.

The method for obtaining the biosensor prototype for the electrochemical detection of SARS-CoV-2 of the present invention is described in detail below.

The shape of the cell required is designed in cut in PSA (Film 1), and the inlet and outlet openings are designed in cut in PC (Film 2). Film 1 is adhered on the aforementioned glass substrate or electrochemical chip, and Film 2 is aligned on Film 1. The fluidic connectors are adhered on these openings with two-sided tape. **Figure 13A** shows the diagram for the assembly of the cell, and **Figure 13B** shows a photograph of the result of the assembly of a cell design on a commercial electrochemical detection chip.

Once the sample has been injected, plugs are placed and everything is sealed and ready for carrying out the method of the invention. Once the reaction has finished and the result has been measured, the chip is disposed of.

The proposed cell design perfectly houses the 20 µl of sample injected in each determination. Likewise, the filling of the biosensors of the invention through the inlet port has been assayed using a pipette, and it did not give rise to bubbles or dead volumes.

**Figure 14** shows the two voltammetries obtained for each of the samples assayed. It can be observed that the redox potential obtained is what is expected for methylene blue, and, therefore, that the reaction being studied is the indicated reaction. Furthermore, the decrease in oxidation peak intensity is evident in the case of the positive control sample (Vircell), pointing out that, indeed, part of the methylene blue of the medium has been consumed during the LAMP isothermal amplification reaction, and, therefore, the presence of SARS-CoV-2 in the positive sample is confirmed by means of the electrochemical detection method of the present invention.

Additionally, **Figure 15** includes the average values and standard deviations for the three repetitions assayed for each of the positive and negative control samples. As can be observed in the present figure, there are important differences in electrochemical current intensity between the positive sample and negative sample. Thus, negative samples have an intensity of at least 2.77 times higher on average than positive samples. The reduction of the current observed in positive samples is related to the intercalation of methylene blue in the new copies generated during the LAMP isothermal amplification process.

The results obtained in the present example demonstrate that by means of the electrochemical detection method of the present invention, it is possible to discriminate between samples that are positive and negative for SARS-CoV-2.

### EXAMPLE 3. DETERMINATION OF THE LIMIT OF DETECTION.

For the limit of detection study for each of the regions ND1-1 (SEQ ID NO: 1), NDB3 (SEQ ID NO: 2), S.45 (SEQ ID NO: 3), ORF1ab.99 (SEQ ID NO: 4) and E.105 (SEQ ID NO: 5) of SARS-CoV-2 used in the present invention, with their corresponding sets of primers described, dilutions of calibrated SARS-CoV-2 RNA, i.e., a commercial RNA in respect of which the copies of viral RNA it comprises (Amplirun^{®} Total Sars-Cov-2 Control, Vircell) are known, were performed. 1/2 dilutions or less were being made, starting from 1,000 copies until reaching 16 total RNA copies in the amplification reaction for each of the aforementioned regions. The detection assays were performed monitoring the reaction in real time in the fluorescent spectrum using ten replicas containing between 10³ and 10¹ copies/reaction, per each of the amounts of RNA assayed. The limit of detection is defined as the concentration for which at least 95 % of the replicas are detected as positive.

Next, the results of the present example for all the amplification regions described in the present invention are described in detail.

### Region ND1-1 (SEQ ID NO: 1) with the set of LAMP primers of SEQ ID NOs: 6 to 11

**Table 16. Table of results corresponding to the limit of detection study which expresses the Cq value, the mean data and the positivity percentage of all measurements in the ten replicas analysed per each quantified level of RNA for the region of primers ND1-1. NEG: negative.**

| **RNA Copies** | **Cq1** | **Cq2** | **Cq3** | **Cq4** | **Cq5** | **Cq6** | **Cq7** | **Cq8** | **Cq9** | **Cq10** | **X** | % |
|---|---|---|---|---|---|---|---|---|---|---|---|---|
| **1000** | 11.6 | 11.5 | 12.4 | 11.7 | 11.3 | 10.8 | 12.2 | 11.1 | 11.1 | 11.2 | 11.5 | 100 |
| **500** | 11.3 | 12.2 | 12.2 | 12.2 | 10.9 | 11.7 | 12.2 | 12.0 | 11.7 | 12.1 | 11.9 | 100 |
| **250** | 12. | 13.1 | 13.3 | 12.2 | 14.1 | 12.6 | 12.3 | 13.5 | 12.5 | 11.1 | 12.7 | 100 |
| **125** | 13.2 | 13.0 | 14.0 | 13.1 | 15.9 | 11.9 | 13.3 | 12.5 | 12.1 | 15.1 | 13.4 | 100 |
| **62** | 13.3 | NEG | 13.3 | 15.5 | 13.4 | 12.5 | 14.8 | 14.1 | 14.7 | 13.6 | 13.9 | 90 |
| **41** | NEG | 16.1 | NEG | 19.3 | 14.2 | 14.5 | 14.6 | 13.0 | 15.7 | 16.8 | 15.5 | 80 |
| **31** | 15.1 | 14.4 | NEG | 13.2 | 18.7 | NEG | 14.5 | N/A | 16.8 | NEG | 15.5 | 70 |
| **16** | 17.2 | 18.5 | 16.1 | NEG | NEG | 15.2 | 22.7 | 19.2 | NEG | NEG | 18.2 | 70 |
| NC | NEG | NEG | NEG | NEG | NEG | NEG | NEG | NEG | NEG | NEG | - | |

The data shown in **Table 16** is represented in the graph shown in **Figure 16****.** As can be observed in said **Figure 16****,** the limit of detection reached with a 95 % confidence level is 100 copies per reaction for region ND1-1 (SEQ ID NO: 1) and 62 copies per reaction with a 90 % confidence level.

### Region ND3B (SEQ ID NO: 2) with the set of LAMP primers of SEQ ID NOs: 12 to 17

**Table 17. Table of results corresponding to the limit of detection study which expresses the Cq value, the mean data and the positivity percentage of all measurements in the ten replicas analysed per each quantified level of RNA for the region of primers ND3B. NEG: negative**

| **RNA Copies** | **Cq1** | **Cq2** | **Cq3** | **Cq4** | **Cq5** | **Cq6** | **Cq7** | **Cq8** | **Cq9** | **Cq10** | **X** | **%** |
|---|---|---|---|---|---|---|---|---|---|---|---|---|
| **1000** | 12.5 | 13.6 | 13.0 | 12.3 | 14.2 | 12.5 | 12.9 | 13.4 | 12.7 | 12.3 | 11.5 | 100 |
| **500** | 14.7 | 14.8 | 14.3 | 15.1 | 15.9 | 13.5 | 12.2 | 14.9 | 13.6 | 13.0 | 11.9 | 100 |
| **250** | 14.0 | 15.6 | 16.3 | 14.6 | 15.4 | 14.4 | 14.4 | 15.4 | 18.0 | 13.2 | 12.7 | 100 |
| **125** | 30.5 | 16.7 | 13.5 | 15.9 | 16.1 | 30.1 | 20.8 | 14.2 | 27.5 | 17.5 | 13.4 | 100 |
| **62** | 19.6 | 17.0 | 17.9 | 17.2 | 16.9 | 18.3 | 15.2 | 20.0 | 14.6 | 16.7 | 13.9 | 100 |
| **41** | 33.2 | NEG | 18.3 | 22.5 | NEG | 20.8 | 16.4 | 16.5 | 18.4 | 16.3 | 15.5 | 80 |
| **31** | 23.0 | 16.9 | NEG | 18.6 | 18.2 | 15.6 | 36.3 | NEG | 21.1 | 19.0 | 15.5 | 80 |
| **16** | 20.2 | NEG | 34.0 | - | 18.3 | NEG | NEG | 19.6 | NEG | 35.3 | 18.2 | 50 |
| NC | NEG | NEG | NEG | NEG | NEG | NEG | NEG | NEG | NEG | NEG | - | |

The data shown in **Table 17** is represented in the graph shown in **Figure 17****.** As can be observed in said **Figure 17****,** the limit of detection reached with a 95 % confidence level is 60 copies per reaction for region ND3B (SEQ ID NO: 2) and 50 copies per reaction with a 90 % confidence level.

### Region S.45 (SEQ ID NO: 3) with the set of LAMP primers of SEQ ID NOs: 18 to 23

**Table 18. Table of results corresponding to the limit of detection study which expresses the Cq value, the mean data and the positivity percentage of all measurements in the ten replicas analysed per each quantified level of RNA for the region of primers S.45. NEG: negative**

| **RNA Copies** | **Cq1** | **Cq2** | **Cq3** | **Cq4** | **Cq5** | **Cq6** | **Cq7** | **Cq8** | **Cq9** | **Cq10** | **X** | **%** |
|---|---|---|---|---|---|---|---|---|---|---|---|---|
| **1000** | 13.4 | 13.7 | 13.7 | 12.8 | 13.3 | 12.8 | 12.4 | 12.6 | 12.7 | 12.5 | 13.0 | 100 |
| **500** | 14.3 | 12.9 | 12.7 | 15.6 | 13.5 | 13.3 | 13.1 | 15.2 | 14.5 | 13.2 | 13.8 | 100 |
| **250** | 15.0 | 13.5 | 14.8 | 14.3 | 15.0 | 15.0 | 12.7 | 14.9 | 14.1 | 13.8 | 14.3 | 100 |
| **125** | 39.4 | 15.1 | 15.7 | 13.5 | 14.8 | 15.8 | 13.8 | 14.1 | 18.9 | 15.9 | 18.0 | 100 |
| **62** | 13.6 | 15.4 | 19.4 | 37.3 | 15.1 | 14.5 | 18.1 | 13.6 | 14.4 | 18.3 | 18.0 | 100 |
| **41** | NEG | 19.3 | 15.0 | NEG | 18.4 | 20.7 | 14.3 | NEG | NEG | 16.2 | 17.3 | 60 |
| **31** | 24.6 | 19.0 | 16.7 | 17.9 | - | 16.3 | - | - | 17.9 | - | 18.7 | 60 |
| **16** | 15.6 | - | 16.3 | - | - | - | - | - | 33.1 | 15.2 | 20.0 | 40 |
| **NC** | - | - | - | - | - | - | - | - | - | - | - | |

The data shown in **Table 18** is represented in the graph shown in **Figure 18****.** As can be observed in said **Figure 18****,** the limit of detection reached with a 95 % confidence level is 60 copies per reaction for region S.45 (SEQ ID NO: 3) and 55 copies per reaction with a 90 % confidence level.

### Region ORF1ab.99 (SEQ ID NO: 4) with the set of LAMP primers of SEQ ID NOs: 24 to 29

**Table 19. Table of results corresponding to the limit of detection study which expresses the Cq value, the mean data and the positivity percentage of all measurements in the ten replicas analysed per each quantified level of RNA for the region of primers ORF1ab.99. NEG: negative**

| **RNA Copies** | **Cq1** | **Cq2** | **Cq3** | **Cq4** | **Cq5** | **Cq6** | **Cq7** | **Cq8** | **Cq9** | **Cq10** | **X** | % |
|---|---|---|---|---|---|---|---|---|---|---|---|---|
| **1000** | 14.6 | 15.2 | 13.7 | 14.4 | 17.4 | 15.6 | 12.9 | 16.1 | 16.1 | 14.1 | 15.0 | 100 |
| **500** | 17.2 | 13.9 | 18.9 | 15.4 | 28.6 | 17.9 | 15.9 | 13.6 | 18.5 | 17.7 | 17.8 | 100 |
| **250** | 27.6 | 14.2 | 15.3 | 14.1 | 12.8 | 31.7 | 16.3 | 13.1 | 13.8 | 14.2 | 17.3 | 100 |
| **125** | 13.7 | 23.2 | 13.6 | 18.2 | 11.9 | - | 15.2 | 15.6 | 15.4 | 14.1 | 15.7 | 90 |
| **100** | 14.0 | NEG | 14.3 | 13.1 | 12.0 | 18.4 | 13.1 | 12.8 | 12.4 | 19.7 | 14.4 | 90 |
| **75** | NEG | NEG | 13.9 | 20.5 | 24.7 | 22.4 | 25.6 | 25.6 | NEG | 16.6 | 21.3 | 70 |
| **62** | NEG | NEG | 21.6 | 24.6 | NEG | 37.1 | NEG | 29.7 | 21.4 | NEG | 26.9 | 50 |
| **31** | NEG | NEG | NEG | 25.5 | NEG | NEG | NEG | 19.1 | NEG | NEG | 22.3 | 20 |
| **NC** | NEG | NEG | NEG | NEG | NEG | NEG | NEG | NEG | NEG | NEG | - | |

The data shown in **Table 19** is represented in the graph shown in **Figure 19****.** As can be observed in said **Figure 19****,** the limit of detection reached with a 95 % confidence level is 200 copies per reaction for region ORF1ab.99 (SEQ ID NO: 4) and 100 copies per reaction with a 90 % confidence level.

### Region E. 105 (SEQ ID NO: 5) with the set of LAMP primers of SEQ ID NOs: 30 to 35

**Table 20. Table of results corresponding to the limit of detection study which expresses the Cq value, the mean data and the positivity percentage of all measurements in the ten replicas analysed per each quantified level of RNA for the region of primers E. 105. NEG: negative**

| **RNA Copies** | **Cq1** | **Cq2** | **Cq3** | **Cq4** | **Cq5** | **Cq6** | **Cq7** | **Cq8** | **Cq9** | **Cq10** | **X** | **%** |
|---|---|---|---|---|---|---|---|---|---|---|---|---|
| **1000** | 13.6 | 14.9 | 15.6 | 15.6 | 14.3 | 15.3 | 14.9 | 16.0 | 13.2 | 20.3 | 15.4 | 100 |
| **500** | 15.8 | 13.6 | 14.0 | 14.8 | 16.7 | 18.3 | 13.2 | 15.4 | 14.9 | 14.9 | 15.1 | 100 |
| **250** | 13.6 | 12.3 | 13.8 | 25.1 | 32.6 | 14.1 | 13.3 | 13.1 | 15.7 | 15.0 | 16.9 | 100 |
| **125** | 14.7 | 17.0 | 20.2 | 13.7 | 26.6 | NEG | 13.2 | 18.1 | 14.2 | 12.7 | 16.7 | 90 |
| **100** | 34.5 | 12.3 | 29.3 | NEG | 38.3 | NEG | 17.1 | NEG | 15.2 | 18.8 | 23.6 | 70 |
| **75** | 39.1 | 26.4 | 18.4 | 36.9 | 25.8 | 22.7 | 16.2 | 16.5 | NEG | 22.0 | 24.9 | 90 |
| **62** | NEG | NEG | 26.6 | 16.9 | 18.5 | 16.8 | 22.3 | NEG | NEG | NEG | 20.2 | 50 |
| **31** | 21.4 | NEG | NEG | 19.9 | 23.4 | NEG | NEG | NEG | NEG | NEG | 21.6 | 30 |
| **NC** | NEG | NEG | NEG | NEG | NEG | NEG | NEG | NEG | NEG | NEG | - | |

The data shown in **Table 20** is represented in the graph shown in **Figure 20****.** As can be observed in said **Figure 20****,** the limit of detection reached with a 95 % confidence level is 200 copies per reaction for region E.105 (SEQ ID NO: 5) and 125 copies per reaction with a 90 % confidence level.

Next, **Table 21** shows the limits of detection reached for each of the regions of SEQ ID NOs 1 to 5 detected by means of the method of the present example.

**Table 21. Limits of detection obtained for each of the sets of primers designed for amplifying regions ND1-1 of SEQ ID NO: 1, ND3B of SEQ ID NO: 2, S.45 of SEQ ID NO: 3, ORF1ab.99 of SEQ ID NO: 4 and E.105 of SEQ ID NO: 5 of the SARS CoV-2 virus.**

| | **Limit of detection 95 % (no. of copies)** | **Limit of detection 90 % (no. of copies)** |
|---|---|---|
| **Region ND1-1 (SEQ ID NO: 1)** | 100 | 62 |
| **Region ND3B (SEQ ID NO: 2)** | 60 | 50 |
| **Region S.45 (SEQ ID NO: 3)** | 60 | 55 |
| **Region ORF1ab.99 (SEQ ID NO: 4)** | 200 | 100 |
| **Region E.105 (SEQ ID NO: 5)** | 200 | 125 |

### EXAMPLE 4. DETECTION OF SARS-CoV-2 IN SAMPLES BY MEANS OF THE METHOD OF THE PRESENT INVENTION WITH OPTICAL DETECTION.

*Detection of SARS-CoV-2 by means of the method of the invention in samples from symptomatic patients by means of the extraction method for extracting nucleic acid by means of Option A and Option B.*

In this part of the study, 10 µL of nasopharyngeal clinical sample coded as B-12/C-168855 and previously diagnosed as negative by the Basque Biobank were assayed. The biological samples and associated data of the subjects included in the present invention were collected, processed and transferred by the Basque Biobank/Bizkaia BioCrossing Node www.biobancovasco.org following standard operating procedures and with the approval of the Ethics Committee and the Scientific Committee.

The RNA extraction step was carried out by means of the rapid extraction method for the detection of symptomatic subjects by means of Option A, as described above in the present document. Briefly:
1. Dispensing the clinical sample in a tube with the Quick Extract product (Lucigen) at a 1:1 ratio and mixing.
2. Incubating for 5 min at 95 °C.
3. Adding a volume to the amplification mixture, which in the particular case of the present example is the amplification mixture comprising the primers capable of detecting specific region S.45 (SEQ ID NO: 3) of SARS-CoV-2, and
4. Reading and detecting the presence of the amplification products in specific region S.45 (SEQ ID NO: 3) by means of the method of the present invention for optical detection.

Viral RNA at a concentration of 500 viral copies per microlitre (5×10² viral copies/µL) (doped sample) was added to the clinical sample assayed, and the results obtained by means of the method of the present invention exceeded the sensitivity previously reported by Ladha *et al.,* 2020 (Ladha A, et al. medRxiv. 2020;8-10), detecting 1×10⁴ viral copies/µl by means of the RT-qPCR method.

The optical detection method of the present invention detects 83 viral copies per reaction by analysing for the clinical sample according to the method detailed, exceeding the limit of detection reached by Lalli MA, *et al.* 2020 (Lalli MA, et al. Clin Chem. 2021;67(2):415-2410) of 100 viral copies per reaction by the colorimetric LAMP method marketed by New England Biolabs (WarmStart Colorimetric LAMP 2X Master Mix).

The results are shown in **Table 22,** where both 2 µl (166 viral copies) and 1 µl of extract of the sample (83 viral copies) are detected.

**Table 22. Results of the detection of region S.45 (SEQ ID NO: 3) comprising the set of LAMP primers of SEQ ID NOs: 18 to 23, in clinical sample B-12/C-168855 from symptomatic patient, performed in triplicate (the Cq value of all the measurements obtained is shown), and to which there have been added 500 viral copies/µl by means of the method for optical detection of the present invention. PC: positive control with 100 viral copies; NC: negative control absence of viral RNA.**

| **Samples** | **Viral RNA copies/assay** | **Volume extract** | **Cq1** | **Cq2** | **Cq3** |
|---|---|---|---|---|---|
| **PC** | 100 | Not extracted | 16.06 | - | - |
| ***B-12*/*C-168855*** | 83 | 1 µl | 14.55 | 16.76 | 13.10 |
| ***B-12*/*C-168855*** | 166 | 2 µl | 14.26 | 14.09 | 17.23 |
| **NC** | 0 | | NEG | - | - |

For the purpose of determining the repeatability of the assay for the same clinical sample on different days and for two other different samples, following the same extraction method assaying 2 µl of the extracts in triplicate by performing LAMP amplification for region ND3B (SEQ ID NO: 2). The results are shown in **Table 23.**

**Table 23. Results of the detection of region ND3B (SEQ ID NO: 2) comprising the set of LAMP primers of SEQ ID NOs: 12 to 17, in three clinical samples from symptomatic patients, performed in triplicate (the Cq value of all the measurements obtained is shown), and to which there have been added 500 viral copies/µl by means of the method for optical detection of the present invention. PC: positive control with 100 viral copies; NC: negative control absence of viral RNA.**

| **Samples** | **Viral RNA copies/assay** | **Volume extract** | **Cq1** | **Cq2** | **Cq3** |
|---|---|---|---|---|---|
| **PC** | 100 | Not extracted | 13.19 | | |
| ***B-12*/*C-168855*** | 166 | 2 µl | 13.32 | 14.35 | 17.14 |
| ***B-12*/*C-168869*** | 166 | 2 µl | 17.38 | 17.17 | 16.11 |
| ***B-12*/*C-168864*** | 166 | 2 µl | 17.56 | 17.65 | 18.51 |
| **NC** | 0 | | - | - | - |

To demonstrate that the electrochemical detection method for the detection of SARS-CoV-2 described in the present invention is likewise effective regardless of the extraction method for extracting the nucleic acid from the isolated biological sample, the same assay described above was carried out with the same clinical samples, but instead of carrying out the extraction of nucleic acid by means of Option A, Option B is now tested, which option does not comprise the step of isolating the nucleic acid from the isolated biological sample analysed and which has been described above in the present document. Briefly, a volume of the sample (5 µL) without prior treatment is added to the amplification solution, which in the particular case of the present example is the amplification mixture comprising the primers capable of detecting specific region S.45 (SEQ ID NO: 3) of SARS-CoV-2. The sample was doped with different amounts of viral RNA at a concentration of 50 viral copies/µl of sample, 25 viral copies/µL and 12 viral copies/ml.

With the present methodology, the method of the present invention detects 62 viral copies/µL. The results are shown in **Table 24.**

**Table 24. Results of the detection for region S.45 (SEQ ID NO: 3) comprising the set of LAMP primers of SEQ ID NOs: 18 to 23, in clinical sample B-12/C-168855 from symptomatic patient, performed in triplicate (the Cq value of all the measurements obtained is shown), and to which there have been added different concentrations of viral copies/µL by means of the method for optical detection of the present invention. PC1: positive control with 125 viral copies/µL; PC2: positive control with 62 viral copies/µL; NC: negative control absence of viral RNA. NEG: negative.**

| **Samples** | **RNA (copies /µl sample)** | **Sample volume (µl)** | **Cq1** | **Cq2** | **Cq3** |
|---|---|---|---|---|---|
| **CP1** | 125 | | 15.10 | | |
| **CP2** | 62 | | 16.44 | | |
| **B-12/C-168855** | 50 | 5 | 14.45 | 16.16 | 15.03 |
| **B-12/C-168855** | 25 | 5 | 18 | 13.13 | 16.12 |
| **B-12/C-168855** | 12 | 5 | NEG | - | - |
| **NC** | 0 | | NEG | - | - |

*Detection* of *SARS-CoV-2 by means of the method of the invention for population screening (asymptomatic patients).*

Shown below are the results obtained by means of the method of the present invention when it is used for population screening, and, therefore, the extraction method for extracting nucleic acid is carried out using, preferably, the Applied Biosystems^{™} MagMAX^{™} Viral/Pathogen Nucleic Acid Isolation Kit, and the manufacturer's instructions are followed. Therefore, the following steps were carried out following the manufacturer's protocol, but with slight modifications and optimisations so that the process has an operation that is simpler, more rapid and with a higher extraction yield. This system allows extracting the volume of samples required by the user by scaling the rest of the volumes of the process. The method for an initial sample volume of 20 µl is herein presented, and a clinical sample coded as B-12/C-164853 and previously diagnosed as negative by the Basque Biobank is tested. The LAMP assay was performed with the sets of primers of region S.45 (SEQ ID NO: 3), and the sample was doped with a viral RNA at a concentration of 50 viral copies/µL.
1. Adding 0.5 µl of proteinase K to the sample.
2. Adding 55 µl of the binding bead mix.
3. Inversion 5 times.
4. Incubating for 5 min at 65 °C.
5. Incubating for 5 min at ambient temperature.
6. Placing the tube in the magnetic rack for 1 min. Suctioning and discarding the liquid.
7. Adding for 30 sec 200 µl of Wash Buffer to the sample, keeping it in the magnetic rack. Eliminating the supernatant.
8. Adding for 30 sec 500 µl of 80 % ethanol to the sample, keeping it in the magnetic rack). Eliminating the supernatant.
9. Leaving the nanoparticles to dry for 2 min.
10. Adding 10 µl of PCR-grade water and mixing.
11. Incubating for 5 min at 65 °C.
12. Placing the tube in the magnetic rack for 1 min.
13. Adding a volume of the eluate to the amplification mixture, which in the particular case of the present example is the amplification mixture comprising the primers capable of detecting specific region S.45 (SEQ ID NO: 3) of SARS-CoV-2, and
14. Reading and detecting the presence of the amplification products in specific region S.45 (SEQ ID NO: 3) by means of the method of the present invention for optical detection.

The results obtained are shown in **Table 25**.

**Table 25. Results of the detection of region S.45 (SEQ ID NO: 3) comprising the set of LAMP primers of SEQ ID NOs: 18 to 23, in clinical sample B-12/C-164853 from symptomatic patient, performed in triplicate (the Cq value of all the measurements obtained is shown), and to which there have been added 50 viral copies/µl by means of the method for optical detection of the present invention. PC: positive control with 100 viral copies; NC: negative control absence of viral RNA.**

| **Samples** | **Viral RNA copies/assay** | **Volume extract** | **Cq1** | **Cq2** | **Cq3** |
|---|---|---|---|---|---|
| **PC** | 500 | Not extracted | 13.44 | - | - |
| **B-12/C-164853** | 500 | 5 µl | 13.01 | 15.21 | 15.78 |
| **NC** | 0 | | NEG | - | - |

To demonstrate the efficacy of the optical detection method the optical detection of SARS-CoV-2 of the present invention, comparative experiments have been conducted between the method of the present invention for optical detection and a kit marketed as SARS-CoV-2 Rapid colorimetric LAMP Assay Kit (New England Biolabs) based on a colorimetric change of the LAMP reaction in the presence of protons causing the pH to drop, resulting in the colour changing from pink (negative result for the absence of viral RNA) to yellow (positive result for the presence of viral RNA).

The comparative assays for the specificity study have demonstrated that in addition to detecting the RNA from SARS-CoV-2, the commercial kit of New England Biolabs also detects RNA from SARS 2003 (**Figure 21**). This demonstrates that the New England Biolabs kit is not specific for SARS-CoV-2 and since it also detects SARS2003, it can give rise to false positives. In contrast, the method and kit described in the present invention is specific for SARS-CoV-2 and does not detect other viruses, as has been demonstrated in the examples included in the present document (see Examples 1 and 2).

In addition, the clinical samples analysed above and to which there have been added 500 viral copies/µl (doped samples) with the New England Biolabs kit and with the optical detection methods of the present invention were assayed. The rapid extraction system for rapidly extracting genetic material, described for the detection/diagnosis of symptomatic patients, was used.

The results obtained with the commercial kit (SARS-CoV-2 Rapid colorimetric LAMP Assay Kit) of New England Biolabs are shown in **Figure 22** and **Table 26**.

**Table 26. Results obtained with the New England Biolabs kit performed in triplicate. PC: positive control with 100 viral copies; NC is the negative control of the reaction or absence of viral RNA.**

| **Samples** | **Viral RNA copies/assay** | **Result 1** | **Result 2** | **Result 3** |
|---|---|---|---|---|
| **PC** | 100 | POS (yellow) | | |
| ***B-12*/*C-168855*** | 166 | NEG (pink) | NEG (pink) | NEG (pink) |
| **NC** | 0 | NEG (pink) | | |

As can be observed both in **Figure 22** and in **Table 26**, the clinical sample that contained 166 copies of viral RNA was not detected as positive for SARS-CoV-2 by means of the New England Biolabs kit, giving rise to a false negative result. On the contrary, when said sample was assayed using the optical detection system of the present invention for the fragment of SEQ ID NO: 3 (S.45) of SARS-CoV-2 of the present invention, positive results were obtained for same both in the visible spectrum and in the ultraviolet spectrum (**Figure 23** and **Table 27**).

**Table 27. Results obtained by means of the optical detection method of the present invention performed in triplicate for region S.45 (SEQ ID NO: 3). PC: positive control with 100 viral copies; NC is the negative control of the reaction or absence of viral RNA.**

| **Samples** | **Tubes** | **Viral RNA copies/assay** | **Extract volume** | **Result 1** | **Result 2** | **Result 3** |
|---|---|---|---|---|---|---|
| **PC** | 1 | 100 | Not extracted | POS (yellow) | | |
| ***B-12*/*C-168855*** | 2-4 | 83 | 1 µl | POS (yellow) | POS (yellow) | POS (yellow) |
| ***B-12*/*C-168855*** | 5-7 | 166 | 2 µl | POS (yellow) | POS (yellow) | POS (yellow) |
| **NC** | 8 | 0 | Not extracted | NEG (orange) | | |

Therefore, the results shown in the present document clearly show that the methods described in the present invention reach a high sensitivity detecting up to 62 copies per reaction and, surprisingly, reach a specificity of 100 % with all the exclusive viruses assayed and also in the studies performed *in silico.* These sensitivity and specificity values are surprisingly better with respect to other studies known in the state of the art also relating to the colorimetric determination of SARS CoV-2 by means of RT-LAMP. Thus, for example, the group of dos Santos *et al.,* (dos Santos et al., J Braz Chem Soc. 2021; 32 (11):2071-2077) reach in their study a limit of detection of 300 copies per reaction, with said limit being much higher than the one obtained by means of the use of the methods described in the present invention.

Furthermore, the set of primers by the group of Park *et al.* (Park et al., J Mol Diagn. 2020 Jun; 22(6): 729-735) and Baek *et al.,* (Baek et al., Emerg Microbes Infect. 2020 Dec;9(1):998-1007) have a specificity of 100 % with respect to the assayed viruses, just like the methods described in the present invention, but the limit of detection is higher, being 100 copies per reaction, compared to the 62 copies reached and demonstrated in the examples included in the present document.

Likewise, the group of Huang *et al.* (Huang et al. Microb Biotechnol. 2020 Jul;13(4):950-961), describes obtaining a lower limit of 2 copies per reaction, which improves that reached and demonstrated in the present invention, but with the difference that Huang *et al.* do not perform specificity studies, solo but rather only confirm that the LAMP reaction described in said paper does not detect human DNA, but they do not experimentally check its specificity for other viruses. The same occurs in the study by Yoshikawa et al. (PLOS Neglected Tropical Diseases. 2020; 14(11): e0008855), where it is described that the limit reached is 50 copies per reaction, but specificity assays are only performed for a single virus. In addition, in Yoshikawa *et al.,* the detection is not done by means of a change in colour but rather by measuring the fluorescence, requiring more sophisticated equipment.

In addition to all the above, the colorimetric kit used in the cases previously mentioned is the colorimetric kit marketed by New England Biolabs, with the exception of the one used by Park *et al.* who do not demonstrate the efficiency of the test conducted with clinical samples since their paper only presents results obtained with purified viral RNA without the interference of the biological sample. The rest of the publications use, as mentioned, the New England Biolabs colorimetric kit based on a change in pH which, as experimentally demonstrated, presents problems in changing colour when the viral RNA extracted from clinical samples by means of rapid techniques compatible with POC systems is analysed, the pH of the samples themselves interferes with the results.

In contrast with the methods, LAMP primers, amplification solutions and reading reagents, as well as with the kits developed for carrying out the methods of the present

## Claims

1. An *in vitro*/*ex vivo* method for the detection of the SARS-CoV-2 virus in an isolated biological sample, wherein the method comprises the steps of
a) extracting genetic material from the isolated biological sample;
b) performing loop-mediated isothermal amplification (LAMP) of the genetic material isolated in step (a), with a reading reagent comprising at least one amplification solution, said amplification solution comprises a set of LAMP primers for amplifying at least two specific regions of the SARS-CoV-2 virus selected from: region ND3B (SEQ ID NO: 2) and region S.45 (SEQ ID NO: 3);
wherein the set of LAMP primers for amplifying region ND3B (SEQ ID NO: 2) comprises:
- a forward primer F3 comprising a nucleotide sequence of SEQ ID NO: 12,
- a backward primer B3 comprising a nucleotide sequence of SEQ ID NO: 13,
- a forward inner primer FIP comprising a nucleotide sequence of SEQ ID NO: 14,
- a backward inner primer BIP comprising a nucleotide sequence of SEQ ID NO: 15,
- a forward loop primer LF comprising a nucleotide sequence of SEQ ID NO: 16; and
- a backward loop primer LB comprising a nucleotide sequence of SEQ ID NO: 17;
and wherein the set of LAMP primers for amplifying region S.45 (SEQ ID NO: 3) comprises:
- a forward primer F3 comprising a nucleotide sequence of SEQ ID NO: 18,
- a backward primer B3 comprising a nucleotide sequence of SEQ ID NO: 19,
- a forward inner primer FIP comprising a nucleotide sequence of SEQ ID NO. 20,
- a backward inner primer BIP comprising a nucleotide sequence of SEQ ID NO: 21,
- a forward loop primer LF comprising a nucleotide sequence of SEQ ID NO: 22, and
- a backward loop primer LB comprising a nucleotide sequence of SEQ ID NO: 23,
c) reading and detecting the presence or absence of an amplification product of the specific regions of step b) by means of an optical and/or electrochemical method.

2. An *in vitro*/*ex vivo* method for the diagnosis of the presence of SARS-CoV-2 in an isolated biological sample, wherein the method comprises the steps of
a) extracting genetic material from the isolated biological sample;
b) performing loop-mediated isothermal amplification (LAMP) of the genetic material isolated in step (a), with a reading reagent comprising at least one amplification solution, said amplification solution comprises a set of LAMP primers for amplifying at least two specific regions of the SARS-CoV-2 virus selected from: region ND3B (SEQ ID NO: 2) and region S.45 (SEQ ID NO: 3);
wherein the set of LAMP primers for amplifying region ND3B (SEQ ID NO: 2) comprises:
- a forward primer F3 comprising a nucleotide sequence of SEQ ID NO: 12,
- a backward primer B3 comprising a nucleotide sequence of SEQ ID NO: 13,
- a forward inner primer FIP comprising a nucleotide sequence of SEQ ID NO: 14,
- a backward inner primer BIP comprising a nucleotide sequence of SEQ ID NO: 15,
- a forward loop primer LF comprising a nucleotide sequence of SEQ ID NO: 16; and
- a backward loop primer LB comprising a nucleotide sequence of SEQ ID NO: 17;
and wherein the set of LAMP primers for amplifying region S.45 (SEQ ID NO: 3) comprises:
- a forward primer F3 comprising a nucleotide sequence of SEQ ID NO: 18,
- a backward primer B3 comprising a nucleotide sequence of SEQ ID NO: 19,
- a forward inner primer FIP comprising a nucleotide sequence of SEQ ID NO. 20,
- a backward inner primer BIP comprising a nucleotide sequence of SEQ ID NO: 21,
- a forward loop primer LF comprising a nucleotide sequence of SEQ ID NO: 22, and
- a backward loop primer LB comprising a nucleotide sequence of SEQ ID NO: 23,
c) reading and detecting the presence or absence of an amplification product of the specific regions of step b) by means of an optical or electrochemical method,
wherein
i. the presence of amplification product for both regions ND3B and S.45 is indicative of the presence of SARS-CoV-2 in the sample, and
ii. the absence of amplification product for both regions ND3B and S.45 is indicative of the absence of SARS-COV-2 in the sample.

3. The method according to any of claims 1 or 2, wherein if in step c) the amplification product is detected, indistinctly, for one of the regions but not for the other region, then a new step b) will be carried out in which the amplification of specific regions of SARS CoV-2 selected from the list consisting of region ND1-1 (SEQ ID NO: 1), region ORF1ab.99 (SEQ ID NO: 3) and/or region E.105 (SEQ ID NO: 5), will additionally be performed, and when the presence of amplification product of at least one of said regions is detected, it is indicative of the presence of SARS-CoV-2 in the sample.

4. The method according to claim 3, wherein the set of LAMP primers for amplifying region ND1-1 (SEQ ID NO: 1) comprises:
- a forward primer F3 comprising a nucleotide sequence of SEQ ID NO: 6,
- a backward primer B3 comprising a nucleotide sequence of SEQ ID NO: 7,
- a forward inner primer FIP comprising a nucleotide sequence of SEQ ID NO. 8,
- a backward inner primer BIP comprising a nucleotide sequence of SEQ ID NO: 9,
- a forward loop primer LF comprising a nucleotide sequence of SEQ ID NO: 10, and
- a backward loop primer LB comprising a nucleotide sequence of SEQ ID NO: 11,
wherein the set of LAMP primers for amplifying region ORF1ab.99 (SEQ ID NO: 4) comprises:
- a forward primer F3 comprising a nucleotide sequence of SEQ ID NO: 24,
- a backward primer B3 comprising a nucleotide sequence of SEQ ID NO: 25,
- a forward inner primer FIP comprising a nucleotide sequence of SEQ ID NO. 26,
- a backward inner primer BIP comprising a nucleotide sequence of SEQ ID NO: 27,
- a forward loop primer LF comprising a nucleotide sequence of SEQ ID NO: 28, and
- a backward loop primer LB comprising a nucleotide sequence of SEQ ID NO: 29,
and wherein the set of LAMP primers for amplifying region E.105 (SEQ ID NO: 5) comprises:
- a forward primer F3 comprising a nucleotide sequence of SEQ ID NO: 30,
- a backward primer B3 comprising a nucleotide sequence of SEQ ID NO: 31,
- a forward inner primer FIP comprising a nucleotide sequence of SEQ ID NO. 32,
- a backward inner primer BIP comprising a nucleotide sequence of SEQ ID NO: 33,
- a forward loop primer LF comprising a nucleotide sequence of SEQ ID NO: 34, and
- a backward loop primer LB comprising a nucleotide sequence of SEQ ID NO: 35.

5. The method according to any of claims 1 to 4, wherein the biological sample is selected from a nasopharyngeal sample or a saliva sample.

6. The method according to any one of claims 1 to 5, wherein the optical detection methods are selected from fluorescent, colorimetric and/or chemiluminescent methods.

7. The method according to any one of claims 1 to 5, wherein the detection method of step (c) is an optical detection method, preferably a fluorescent, colorimetric or chemiluminescent method, and the reading reagent of step (b) comprises a fluorescent dye which is selected from the list consisting of calcein, EvaGreen, Syber Green, hydroxynaphthol blue, neutral red, crystal violet and SYTO 9, preferably calcein and/or EvaGreen.

8. The method according to any one of claims 1 to 5, wherein the detection method of step (c) is an electrochemical detection method, and the reading reagent of step (b) comprises a redox substrate which is selected from the list consisting of: methylene blue, toluidine blue, ferrocene, anthraquinone, osmium complexes, meldola blue, anthraquinone acids, daunomycin, Hoeschst 33258 and [Co(phen)₃](ClO₄)₃, preferably methylene blue.

9. A set of LAMP primers for amplifying at least two of the SARS-CoV-2 viruses selected from: region ND3B (SEQ ID NO: 2) and region S.45 (SEQ ID NO: 3); wherein the set of LAMP primers for amplifying region ND3B (SEQ ID NO: 2) comprises:
- a forward primer F3 comprising a nucleotide sequence of SEQ ID NO: 12,
- a backward primer B3 comprising a nucleotide sequence of SEQ ID NO: 13,
- a forward inner primer FIP comprising a nucleotide sequence of SEQ ID NO: 14,
- a backward inner primer BIP comprising a nucleotide sequence of SEQ ID NO: 15,
- a forward loop primer LF comprising a nucleotide sequence of SEQ ID NO: 16; and
- a backward loop primer LB comprising a nucleotide sequence of SEQ ID NO: 17;
and wherein the set of LAMP primers for amplifying region S.45 (SEQ ID NO: 3) comprises:
- a forward primer F3 comprising a nucleotide sequence of SEQ ID NO: 18,
- a backward primer B3 comprising a nucleotide sequence of SEQ ID NO: 19,
- a forward inner primer FIP comprising a nucleotide sequence of SEQ ID NO. 20,
- a backward inner primer BIP comprising a nucleotide sequence of SEQ ID NO: 21,
- a forward loop primer LF comprising a nucleotide sequence of SEQ ID NO: 22, and
- a backward loop primer LB comprising a nucleotide sequence of SEQ ID NO: 23.

10. The set of LAMP primers according to claim 9, further comprising at least one set of primers for amplifying the specific regions of SARS CoV-2 selected from the list consisting of region ND1-1 (SEQ ID NO: 1), region ORF1ab.99 (SEQ ID NO: 3) and/or region E.105 (SEQ ID NO: 5),
wherein the set of primers for amplifying region ND1-1 (SEQ ID NO: 1), comprises:
- a forward primer F3 comprising a nucleotide sequence of SEQ ID NO: 6,
- a backward primer B3 comprising a nucleotide sequence of SEQ ID NO: 7,
- a forward inner primer FIP comprising a nucleotide sequence of SEQ ID NO. 8,
- a backward inner primer BIP comprising a nucleotide sequence of SEQ ID NO: 9,
- a forward loop primer LF comprising a nucleotide sequence of SEQ ID NO: 10, and
- a backward loop primer LB comprising a nucleotide sequence of SEQ ID NO: 11,
wherein the set of LAMP primers for amplifying region ORF1ab.99 (SEQ ID NO: 3), comprises:
- a forward primer F3 comprising a nucleotide sequence of SEQ ID NO: 24,
- a backward primer B3 comprising a nucleotide sequence of SEQ ID NO: 25,
- a forward inner primer FIP comprising a nucleotide sequence of SEQ ID NO. 26,
- a backward inner primer BIP comprising a nucleotide sequence of SEQ ID NO: 27,
- a forward loop primer LF comprising a nucleotide sequence of SEQ ID NO: 28, and
- a backward loop primer LB comprising a nucleotide sequence of SEQ ID NO: 29,
and wherein the set of LAMP primers for amplifying region E.105 (SEQ ID NO: 5), comprises:
- a forward primer F3 comprising a nucleotide sequence of SEQ ID NO: 30,
- a backward primer B3 comprising a nucleotide sequence of SEQ ID NO: 31,
- a forward inner primer FIP comprising a nucleotide sequence of SEQ ID NO. 32,
- a backward inner primer BIP comprising a nucleotide sequence of SEQ ID NO: 33,
- a forward loop primer LF comprising a nucleotide sequence of SEQ ID NO: 34, and
- a backward loop primer LB comprising a nucleotide sequence of SEQ ID NO: 35.

11. A reading reagent for detecting at least two regions of the SARS-CoV-2 virus selected from: region ND3B (SEQ ID NO: 2) and region S.45 (SEQ ID NO: 3), wherein said reagent comprises at least one amplification solution comprising the set of LAMP primers according to any of claims 9 to 10.

12. The reading reagent according to claim 11, wherein said reagent further comprises at least one optical reading compound or one electrochemical reading compound, preferably wherein the optical reading compound is a fluorescent dye selected from calcein, EvaGreen, Syber Green, hydroxynaphthol blue, neutral red, crystal violet and SYTO 9, preferably calcein and/or EvaGreen, and wherein the electrochemical compound comprises a redox substrate which is selected from the list consisting of: methylene blue, toluidine blue, ferrocene, anthraquinone, osmium complexes, meldola blue, anthraquinone acids, daunomycin, Hoeschst 33258 and [Co(phen)₃](ClO₄)₃, preferably methylene blue.

13. A kit for the detection of the SARS-CoV-2 virus in an isolated biological sample, wherein the kit comprises reagents for amplifying the genetic material in said sample using a LAMP technique, and the set of LAMP primers according to any one of claims 9 to 10, or the reading reagent according to claims 11 to 12.

14. Use of the set of LAMP primers according to any one of claims 9 to 10, of the reading reagent according to any one of claims 11 to 12, or of the kit according to claim 13, for detecting the presence of the SARS-CoV-2 virus and/or for the diagnosis of the SARS-CoV-2 virus, in an isolated biological sample.
